(19) Europäisches Patentamt
European Patent Office
Office européen des brevets

(11) **EP 2 995 289 A1**

(12) **EUROPEAN PATENT APPLICATION**

(43) Date of publication:
**16.03.2016  Bulletin 2016/11**

(21) Application number: **14184265.8**

(22) Date of filing: **10.09.2014**

(51) Int Cl.:
*A61F 13/49* (2006.01)         *A61F 13/494* (2006.01)
*A61F 13/56* (2006.01)         *A61F 13/00* (2006.01)
*B32B 5/04* (2006.01)          *B32B 7/00* (2006.01)
*B32B 7/04* (2006.01)          *A61F 13/15* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **The Procter & Gamble Company
Cincinnati, OH 45202 (US)**

(72) Inventors:
• **Liebe, Tina
65824 Schwalbach am Taunus (DE)**

• **Hippe, Matthias Konrad
65824 Schwalbach am Taunus (DE)**
• **Kline, Mark
Okeana, OH Ohio 45053 (US)**

(74) Representative: **Heide, Ute
Procter & Gamble Service GmbH
IP Department
Frankfurter Strasse 145
61476 Kronberg im Taunus (DE)**

(54) **MULTI-LEVEL CELL FORMING STRUCTURES AND THEIR USE IN DISPOSABLE CONSUMER PRODUCTS**

(57)    The invention refers to a structure which is able to elongate and simultaneously convert from an initial flat configuration into an erected configuration and which comprises multiple levels. The structure may be part of a flexible package or of a disposable absorbent article such as a diaper, a pant or a sanitary napkin.

**Fig. 1a**

**Fig. 1b**

EP 2 995 289 A1

**Description**

BACKGROUND OF THE INVENTION

The use of extensible materials as well as use of elastic materials in a large variety of products, such as absorbent articles, is well known in the art. For example, such materials are often comprised in waistbands, ear panels or leg cuffs of diapers.

A drawback commonly associated with extensible materials and elastic materials, such as (elastic) films or nonwoven webs, is that their width decreases when they are elongated along their lengthwise dimension. This property is typically referred to as necking. Also, extensible as well as elastic materials typically decrease in caliper, i.e. in thickness, when being elongated.

Generally, materials which increase in thickness when being stretched are also known in the art. These so-called "auxetics" are materials which have a negative Poisson's ratio. When stretched, they become thicker perpendicular to the applied force. This behavior is due to their hinge-like structures, which flex when stretched. Auxetic materials can be single molecules or a particular structure of macroscopic matter. Such materials are expected to have mechanical properties such as high energy absorption and fracture resistance. Auxetics have been described as being useful in applications such as body armor, packing material, knee and elbow pads, robust shock absorption material, and sponge mops. Typically, though their thickness increases upon elongation, (macroscopic) auxetic materials have a relatively significant thickness already in their relaxed state. That is, known auxetic structures are typically non-flat structures having predominantly 3-dimensional shape when they are in their relaxed state.

The general use of auxetic materials in absorbent articles, such as diapers, has been disclosed in WO 2007/046069 A1 "Absorbent article comprising auxetic materials".

There is still a need for extensible structures which increase in caliper when being stretched. Further, it would be desirable that these structures show auxetic behavior in that they increase in caliper (i.e. thickness) upon being stretched, while the structures should desirably be relatively flat in their initial, non-stretched state.

Such structures may also exhibit elastic-like behavior such that they can retract to substantially their initial shape when an applied force, upon which the structure is converted them into an elongated shape with increased caliper, is removed. Alternatively, the structures may be facilitated such that the structure, once elongated, tends to remain substantially in its elongated configuration with increased caliper when the applied force is removed. Also, structures may convert to an intermediate configuration when the applied force is removed.

It would also be desirable to be able to make such structures from relatively inexpensive, widely available feedstock materials.

It would also be desirable to provide structures which can be adapted and tailed for many different applications and needs.

Such structures would have wide applicability, for example in disposable consumer products, such as absorbent articles (e.g. diapers), wound dressings, bandages but also in flexible packaging. Especially, a flat configuration in their non-stretched state would make such structures attractive for use in disposable absorbent articles, which are typically densely packed as one or more rows of stacked articles, wherein the individual absorbent article is in a flat, folded configuration.

SUMMARY OF THE INVENTION

The invention refers to a multi-level structure (hereinafter simply referred to as "structure") has a longitudinal dimension and a lateral dimension perpendicular to the longitudinal dimension. The structure comprises at least a first and a second level. The structure has a first outermost layer, a second outermost layer, and at least a first intermediate layer, with all layers having a longitudinal dimension parallel to the longitudinal dimension of the structure and being confined by two spaced apart lateral edges, and a lateral dimension parallel to the lateral dimension of the structure and being confined by two spaced apart longitudinal edges.

The layers at least partly overlap each other; with the at least first intermediate layer being positioned subjacent the first outermost layer and superjacent the second outermost layer.

Each level of the structure is confined in a direction perpendicular to the longitudinal and lateral dimension by one of the layers forming the superjacent layer of the level and further confined by another of the layers forming the subjacent layer of the level.

The first and second outermost layer are able to shift relative to each other in opposite directions along the longitudinal structure dimension upon application of a force along the longitudinal dimension, whereby the structure is elongated along the longitudinal dimension and simultaneously converted from an initial flat configuration into an erected configuration in a direction perpendicular to the longitudinal and the lateral dimension.

Each level in the structure comprises one or more (such as two or more than two) ligaments, each ligament having a first surface and a second surface, a longitudinal dimension confined by two spaced apart lateral ligament edges, and a lateral dimension confined by two spaced apart longitudinal ligament edges. Each ligament is provided between the superjacent layer and the subjacent layer of the respective level and is attached at or adjacent to one of the ligament's lateral edges in a first ligament attachment region to the surface of the superjacent layer which faces towards the subjacent layer of the respective level, and is further attached at or adjacent to the ligament's other lateral edge in a second ligament attachment region to the surface of the subjacent layer which faces towards the superjacent layer of the respective level. The region of each ligament between the first and second ligament attachment region forms a free intermediate portion.

The ligaments in each level are spaced apart from one another along the longitudinal dimension of the structure when the structure is in its erected configuration, and the attachment of all ligaments is such that the free intermediate portions of all ligaments in the structure are able to convert from an initial flat configuration to an erected configuration upon application of a force along the longitudinal dimension of the structure. Thus the structure is converted as a whole from an initial flat configuration into an erected configuration, wherein the erection is in the direction perpendicular to the longitudinal and the lateral dimension of the structure.

When the ligaments are in their erected configuration, each ligament may either adopt a Z-like shape, a C-like shape, a shape forming a T in one of the first or second ligament attachment region and taking a tilted shape in the other of the first or second ligament attachment region (hereinafter simply referred to as "T-like shape"), or a Double-T-like shape. All ligaments in a structure adopting a Z-like shape, irrespective by which level they are comprised, will adopt a Z-like shape with the same orientation. Likewise, all ligaments in a structure adopting a C-like shape, irrespective by which level they are comprised, will adopt a C-like shape with the same orientation when the structure is converted into its erected configuration. Hence, no ligaments in a structure adopting a Z-like shape or a C-like shape will have an inverted configuration versus another ligament in the structure with a Z-like or C-like shape.

The names "Z-like shape", "C-like shape", "T-like shape", and "Double-T-like shape" reflect the appearance and shape of the ligaments when viewed from the side parallel to the lateral direction.

The structure may comprise one or more stop aid(s) which define(s) the maximum shifting of the first outermost layer relative to the second outermost layer along the longitudinal dimension in opposite directions when the force along the longitudinal dimension is applied, wherein the maximum shifting defined by the stop aid is less than the maximum shifting provided by the ligaments in the absence of such stop aid.

In the absence of a stop aid, which may be comprised by the structure or which may be external to the structure but having the same effect as a stop aid comprised by the structure, the first outermost layer would be able to continue shifting relative to the second outermost layer in opposite directions along the longitudinal structure dimension upon continued application of a force along the longitudinal dimension structure when the structure is in its erected configuration. Thereby, the ligaments' free intermediate portion would turn over up to about 180° from their position in the initial flat structure configuration to an erected configuration and into a turned-over flat structure configuration. In its final, turned-over flat structure configuration, it would not be possible to elongate the structure any further along the longitudinal dimension, unless at least the first and second outermost layer -and possibly also the ligaments- are made of extensible or elastic material.

The free intermediate portion of each ligament may either remain unattached to the subjacent and superjacent layer (and to all other layers) or may be releasable attached to the subjacent layer and/or to the superjacent layer. The free intermediate portions of the ligaments may not be attached to each other.

The longitudinal dimension of the free intermediate portion of one or more of the ligaments within a level may or may not differ from the longitudinal dimension of the free intermediate portion of one or more other ligaments within the same level.

The structure may comprise one or more stop aid(s) which define(s) the maximum shifting of the first outermost layer relative to the second outermost layer along the longitudinal dimension in opposite directions when the force along the longitudinal dimension is continued to be applied, wherein the maximum shifting defined by the stop aid is less than the maximum shifting possible in the absence of such stop aid.

The one or more stop aid(s) may be selected from the group consisting of:

a) the first and second outermost layer being attached to each other in at least one attachment region, wherein the at least one layer-on-layer attachment region is provided such that one of the first and second outermost layers has a leeway when the structure is in its initial flat configuration, the leeway may be provided between the layer-on-layer attachment region and the ligament which is closest to the layer-on-layer attachment region; said leeway being able to straighten out and/or extend when the structure is transferred into its erected configuration; and

b) a layer-to-layer stop aid extending from the first outermost layer to the second outermost layer and being attached to the first outermost layer in a first layer-to-layer stop aid attachment region and being further attached to the second outermost layer in a second layer-to-layer stop aid attachment region, wherein the layer-to-layer stop aid is provided with a layer-to-layer stop aid leeway between the first layer-to-layer stop aid attachment region and the second layer-to-layer stop aid attachment region when the structure is in its initial flat configuration, said leeway being able to straighten out and/or extend when the structure is transferred into its erected configuration; and

c) a layer-to-ligament stop aid extending from the first or second outermost layer to one of the ligaments comprised by a level which is farthest away from the respective first or second outermost layer, and being attached to the first or second outermost layer in a first layer-to-ligament stop aid attachment region and being attached to the ligament in a second layer-to-ligament stop aid attachment region, wherein the layer-to-ligament stop aid is provided with a layer-to-ligament stop aid leeway between the first layer-to-ligament stop aid attachment region and the second layer-to-ligament stop aid attachment region when the structure is in its initial flat configuration, said leeway being able to straighten out and/or extend when the structure is transferred into its erected configuration; and

d) an enveloping stop aid encircling a least a portion of the first and second outermost layer, of the intermediate layer(s) and of the ligaments between the first and second outermost layer, wherein the enveloping stop aid is attached to the first outermost layer, the second outermost layer, one or more of the intermediate layer(s) and/or one or more ligaments in at least one enveloping stop aid attachment region and wherein the enveloping stop aid is further attached to itself to form a closed loop with a defined circumference around a least a portion of the first and second outermost layer, wherein the circumference of the enveloping stop aid defines the maximum caliper of the structure in its erected configuration; wherein the caliper is perpendicular to the lateral and longitudinal dimension of the structure; and

e) any combinations of a) to d).

The first and second outermost layers and the intermediate layer(s) of the structure may be non-elastic, preferably non elastic and non-extensible at least outside the areas which are provided as leeways comprised by a stop aid. The first and second outermost layers, the intermediate layer(s) and/or the ligaments may be made of film, nonwoven material, paper, sheet-like foam, woven fabric, knitted fabric or combinations thereof

The structure may have a modulus of from about 0.01 N/mm$^2$ to about 0.3 N/mm$^2$ (this modulus range may apply to all areas where the ligaments are located as well as to all areas between neighboring ligaments, hence, all areas of the structure may have a modulus of from about 0.01 N/mm$^2$ to about 0.3 N/mm$^2$).

The longitudinal dimension of the ligaments in the initial flat configuration of structure may be substantially or fully parallel with the longitudinal dimension of the first and second outermost layer and with the intermediate layer(s).

The erected structure configuration, upon release of the force applied along the longitudinal dimension, may return substantially completely to its initial flat configuration.

The ligaments may differ from each other in tensile strength and/or in bending stiffness.

In one or more ligaments, those portions of the free intermediate portion which are directly adjacent to the first and/or second ligament attachment region may have a different bending stiffness and/or different tensile strength than the remaining free intermediate portion of said ligament.

The multilevel structures described herein may be comprised by the absorbent article by one or more of: a front waist feature, a back waist feature, a landing zone, one or two front ears, one or two back ears. The longitudinal dimension of the structure may be substantially parallel to a lateral centerline of the absorbent article and the lateral dimension of the structure may be substantially parallel to the longitudinal centerline of the absorbent article. The multilevel structure may be attached to the absorbent article with areas of the first and second outermost layer at or adjacent to the lateral edges of the multilevel structure.

The multilevel structures of the present invention can be tailored for many different applications to meet are large variety of different needs. For example, the (erected) multilevel structure can be relatively resilient to compression in the Z-direction, thus reliably filling a gap, such as the gluteal groove of a wearer or the gap arising between an absorbent article and the back of a wearer in use when the wearer bends forward. At the same time, the structure can provide sufficient flexibility and pliability to smoothly and comfortably contact the skin of the wearer.

These structures can be comprised by disposable consumer products, such as absorbent articles, e.g. diapers, pants or sanitary napkins. The structures can also be comprised by wound dressings, bandages or in flexible packaging.

BRIEF DESCRIPTION OF THE DRAWINGS

These and other features, aspects and advantages of the present invention will become better understood with regard to the following description, appended claims, and accompanying drawing where:

Fig. 1A is a side view (parallel to the lateral dimension) of an embodiment of the multilevel structure of the present invention- with ligaments in both levels having Z-like shape -, wherein the structure is in its initial flat configuration and wherein the free intermediate portion of all ligaments has the same length

Fig. 1B is a side view of the embodiment of Fig. 1A, wherein the structure is now in its erected configuration

Fig. 2A is a side view (parallel to the lateral dimension) of an embodiment of the multilevel structure of the present invention - with ligaments having Z-like shape -, wherein the structure is in its initial flat configuration and wherein the structure comprises a layer-to-layer stop aid

Fig. 2B is a side view (parallel to the lateral dimension) of the embodiment of Fig 2A, now in its erected configuration

Fig. 3A is a side view (parallel to the lateral dimension) of an embodiment of the multilevel structure of the present invention - with ligaments having Z-like shape -, wherein the structure is in its initial flat configuration and wherein the structure comprises a layer-to-ligament stop aid

Fig. 3B is a side view (parallel to the lateral dimension) of the embodiment of Fig 3A, now in its erected configuration

Fig. 4 is a side view of another embodiment of the multilevel structure of the present invention - with ligaments having Z-like shape and wherein the ligaments are folded when the multilevel structure is in its flat configuration

Fig. 5A is a side view of an embodiment of the multilevel structure of the present invention -with ligaments having C-like shape-, wherein the structure is in its initial flat configuration

Fig. 5B is a side view of the embodiment of Fig. 5A, wherein the structure is in its partly erected configuration

Fig. 5C is a side view of the embodiment of Fig. 5A, wherein the structure is in its maximum erected configuration

Fig. 6A is a side view of an embodiment of the multilevel structure of the present invention -with ligaments having Double-T-like shape-, wherein the structure is in its initial flat configuration

Fig. 6B is a side view of the embodiment of Fig. 6A, wherein the structure is in its partly erected configuration

Fig. 6C is a side view of the embodiment of Fig. 6A, wherein the structure is in its maximum erected configuration

Fig. 7A is a side view of an embodiment of the multilevel structure of the present invention -with ligaments having T-like shape-, wherein the structure is in its initial flat configuration

Fig. 7B is a side view of the embodiment of Fig. 7A, wherein the structure is in its partly erected configuration

Fig. 7C is a side view of the embodiment of Fig. 7A, wherein the structure is in its maximum erected configuration

Fig. 8A is a side view of an embodiment of the multilevel structure of the present invention - with ligaments of Z-like shape having inverse configuration, wherein the structure is in its initial flat configuration

Fig. 8B is a side view of the embodiment of Fig. 8A, wherein the structure is in its erected configuration

Fig. 9A is a side view of an embodiment of the multilevel structure of the present invention - with ligaments of C-like shape having inverse configuration, wherein the structure is in its initial flat configuration

Fig. 9B is a side view of the embodiment of Fig. 9A, wherein the structure is in its erected configuration

Fig. 10 is a side view of an embodiment of a multilevel structure not comprised by the present invention, wherein the structure cannot be converted into an erected configuration

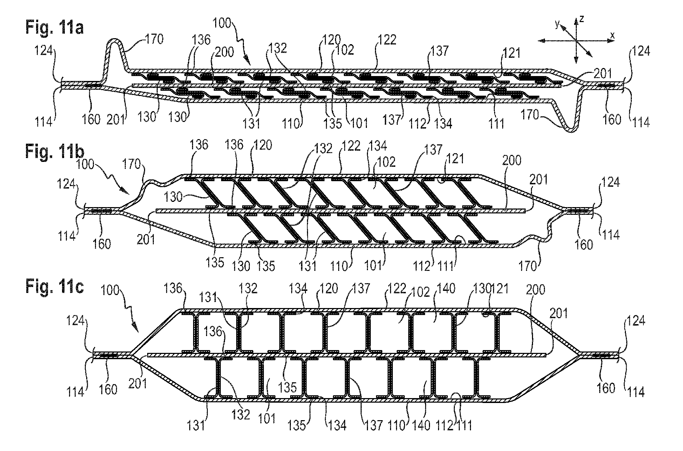

Fig. 11A is a side view of an embodiment of the multilevel structure of the present invention -with ligaments made of two-layered laminates and having Double-T-like shape-, wherein the structure is in its initial flat configuration

Fig. 11B is a side view of the embodiment of Fig. 11A, wherein the structure is in its partly erected configuration

Fig. 11C is a side view of the embodiment of Fig. 11A, wherein the structure is in its maximum erected configuration

Fig. 12A is a side view of another embodiment of the multilevel structure of the present invention -with ligaments compiled of separate pieces of material and having Double-T-like shape-, wherein the structure is in its initial flat configuration

Fig. 12B is a side view of the embodiment of Fig. 12A, wherein the structure is in its partly erected configuration

Fig. 12C is a side view of the embodiment of Fig. 12A, wherein the structure is in its maximum erected configuration

Fig. 13A is a side view of another embodiment of the multilevel structure of the present invention -with ligaments compiled of separate pieces of material and having Z-like shape-, wherein the structure is in its initial flat configuration

Fig. 13B is a side view of the embodiment of Fig. 13A, wherein the structure is in its partly erected configuration

Fig. 13C is a side view of the embodiment of Fig. 13A, wherein the structure is in its maximum erected configuration

Fig. 14A is a side view of another embodiment of the multilevel structure of the present invention -with ligaments having cut out areas and having Z-like shape-, wherein the structure is substantially in its initial flat configuration

Fig. 14B is a side view of the embodiment of Fig. 14A, wherein the structure is in its erected configuration

Fig. 15A is a side view of an embodiment of the multilevel structure of the present invention - with ligaments having Z-like shape- wherein the intermediate layer is attached to the outermost layers and is provided with a leeway in form of a extensible material

Fig. 15B is a side view of the embodiment of Fig. 15A, wherein the structure is in its erected configuration

Fig. 16A is a side view of an embodiment of the multilevel structure of the present invention - with ligaments having Z-like shape- wherein the intermediate layer is attached to the outermost layers and is provided with a leeway in form of a slack

Fig. 16B is a side view of the embodiment of Fig. 16A, wherein the structure is in its erected configuration

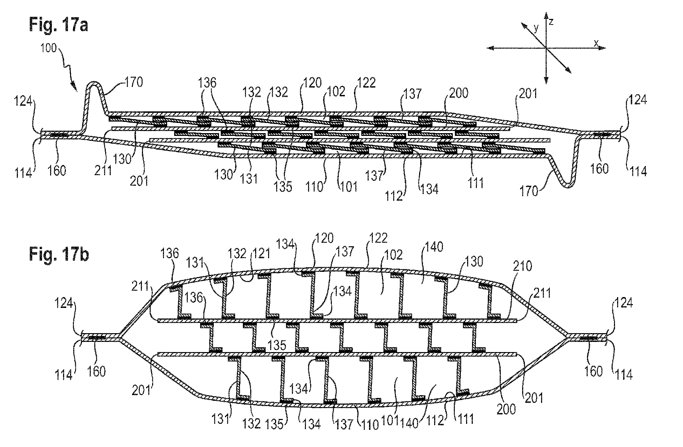

Fig. 17A is a side view of an embodiment of the multilevel structure of the present invention - with ligaments having Z-like shape- wherein the structure has two intermediate layers and three levels

Fig. 17B is a side view of the embodiment of Fig. 17A, wherein the structure is in its erected configuration

Fig. 18A is a side view of an embodiment of the multilevel structure of the present invention - with ligaments having Z-like shape- wherein the intermediate layer is interrupted and consists of two sections

Fig. 18B is a side view of the embodiment of Fig. 18A, wherein the structure is in its erected configuration

Fig. 19 shows a diaper as an exemplary embodiment of an absorbent article, wherein the structure is comprised as a back waistband

Fig. 20 shows a diaper as an exemplary embodiment of an absorbent article, wherein the structure is comprised by the back ears

Fig. 21 is a schematic drawing of parts of the equipment used for the modulus test method

DETAILED DESCRIPTION OF THE INVENTION

Definitions

"Absorbent article" refers to devices that absorb and contain body exudates, and, more specifically, refers to devices that are placed against or in proximity to the body of the wearer to absorb and contain the various exudates discharged from the body. Absorbent articles may include diapers (baby diapers and diapers for adult incontinence), pants, feminine care absorbent articles such as sanitary napkins or pantiliners, breast pads, care mats, bibs, wipes, and the like. As used herein, the term "exudates" includes, but is not limited to, urine, blood, vaginal discharges, breast milk, sweat and fecal matter. Preferred absorbent articles of the present invention are disposable absorbent articles, more preferably disposable diapers and disposable pants.

"Bandage" as used herein, refers to a bandage is a piece of material used either to support a medical device such as wound dressing, or on its own to provide support to the body. Bandages may be used, e.g. during heavy bleeding or following a poisonous bite, in order to slow the flow of blood. Bandages are available in a wide range of types, from generic cloth strips to specialized shaped bandages designed for a specific limb or part of the body. While a wound dressing is in direct contact with a wound, a bandage is not directly in contact with a wound but may be used to support a wound dressing.

"Consumer product" as used herein, refers to an article produced or distributed for sale to a consumer for the personal use, consumption or enjoyment by a consumer in or around a permanent or temporary household or residence. A consumer product is not used in the production of another good. Preferred disposable consumer products of the present invention are absorbent articles, wound dressings and bandages.

"Disposable" is used in its ordinary sense to mean an article that is disposed or discarded after a limited number of usage over varying lengths of time, for example, less than 20 usages, less than 10 usages, less than 5 usages, or less than 2 usages. If the disposable consumer product is a wound dressing or a disposable absorbent article such a diaper, a pant, sanitary napkin, sanitary pad or wet wipe for personal hygiene use, the wound dressing or disposable absorbent article is most often intended to be disposed after single use.

"Diaper" and "pant" refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be preformed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.

The term "film" as used herein refers to a substantially non-fibrous sheet-like material wherein the length and width of the material far exceed the thickness of the material. Typically, films have a thickness of about 0.5 mm or less. Films may be configured to be liquid impermeable and/or vapor permeable (i.e., breathable). Films may be made of polymeric, thermoplastic material, such as polyethylene, polypropylene or the like.

"Non-extensible" as used herein refers to a material which, upon application of a force, elongates beyond its original length by less than 20 % if subjected to the following test: A rectangular piece of the material having a width of 2.54 cm and a length of 25.4 cm is maintained in a vertical position by holding the piece along its upper 2.54 cm wide edge along its complete width. A force of 10 N is applied onto the opposite lower edge along the complete width of the material for 1 minute (at 25°C and 50% rel. humidity; samples should be preconditioned at these temperature and humidity conditions for 2 hours prior to testing). Immediately after one minute, the length of the piece is measured while the force is still applied and the degree of elongation is calculated by subtracting the initial length (25.4 cm) from the length measured after one minute.

If a material elongates beyond its original length by more than 20 % if subjected to the above described test, it is "extensible" as used herein.

"Highly non-extensible" as used herein refers to a material, which, upon application of a force, elongates beyond its original length by less than 10% if subjected to the test described above for "non-extensible" material.

"Non-elastic" as used herein refers to a material which does not recover by more than 20% if subjected to the following test, which is to be carried out immediately subsequent to the test on "non-extensibility" set out above.

Immediately after the length of the rectangular piece of material has been measured while the 10N force is still applied, the force is removed and the piece is laid down flat on a table for 5 minutes (at 25°C and 50% rel. humidity) to be able to recover. Immediately after 5 minutes, the length of the piece is measured again and the degree of elongation is calculated by subtracting the initial length (25.4 cm) from the length after 5 minutes.

The elongation after one minute while the force has been applied (as measured with respect to "non-extensibility") is compared to the elongation after the piece has been laid down flat on a table for 5 minutes: If the elongation does not recover by more than 20%, the material is considered to be "non-elastic".

If a material recovers by more than 20%, the material is considered "elastic" as used herein.

"Highly non-elastic" as used herein refers to a material, which is either "non-extensible" or which does not recover by more than 10% if subjected to the test set out above for "non-elastic".

For use in the cell forming structures of the present invention, extensible, non-extensible, highly non-extensible, elastic, non-elastic and highly non-elastic relate to the dimension of the material, which, once the material has been incorporated into the structure, is parallel to the longitudinal dimension of the structure. Hence, the sample length of 25.4 cm for carrying out the tests described above corresponds to the longitudinal dimension of the cell forming structure once the material has been incorporated into the structure.

A "nonwoven web" is a manufactured web of directionally or randomly oriented fibers, consolidated and bonded together. The term does not include fabrics which are woven, knitted, or stitch-bonded with yarns or filaments. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as meltblowing, spunbonding, solvent spinning, electrospinning, and carding. Nonwoven webs may be bonded by heat and/or pressure or may be adhesively bonded. Bonding may be limited to certain areas of the nonwoven web (point bonding, pattern bonding). Nonwoven webs may also be hydro-entangled or needle-punched. The basis weight of nonwoven fabrics is usually expressed in grams per square meter ($g/m^2$).

A "paper" refers to a wet-formed fibrous structure comprising cellulose fibers.

"Sheet-like foam", as used herein is a solid sheet that is formed by trapping pockets of gas. The solid foam may be closed-cell foam or open-cell foam. In closed-cell foam, the gas forms discrete pockets, each completely surrounded by the solid material. In open-cell foam, the gas pockets connect with each other. "Sheet-like" means that the length and width of the material far exceed the thickness of the material

"Wound dressing", as used herein, is used to cover and protect a wound in order to promote healing and/or prevent further harm.

**Multilevel cell forming structures**

For many applications, such as many applications in absorbent articles or other disposable consumer products, it would be highly desirable to have structures, which are initially flat but which simultaneously increase in caliper (i.e. thickness) when being elongated along their longitudinal dimension.

Moreover, such structures may exhibit an elastic-like behavior, i.e. they are able return -at least to some extent-to their initial longitudinal dimension and also to their initial caliper. Alternatively, the structure may be facilitated such that, once elongated, it remains substantially in its elongated configuration with increased caliper when the applied force is removed. In a still further alternative, structures may convert to an intermediate configuration with a length and caliper in between the initial state and their stretched state when the applied force is removed.

The present invention relates to so-called multilevel cell forming structures (herein referred to simply as "structures") due to the cells formed in each level between neighboring ligaments in the erected structure configuration, the cells being delimited by two neighboring ligaments and a layer forming the superjacent layer of the respective level and further delimited by a layer forming the subjacent layer of the respective level. These structures are initially relatively flat. When a force is applied along the longitudinal dimension (i.e. along the lengthwise extension) of the structure, the structure elongates and simultaneously adopts an erected configuration. Thus, the structure increases in caliper. As used herein, the terms "caliper" and "thickness" are used interchangeably and refer to a direction perpendicular to the lateral and longitudinal dimension. Moreover, when the applied force is released, these structures may be able to revert to substantially their initial flat and shortened configuration. Such structures can be elongated and relaxed repeatedly. It is also possible to put the structure into execution such that the elongated structure does not or only to a certain extent return to its initial flat and shortened configuration when the applied force is released.

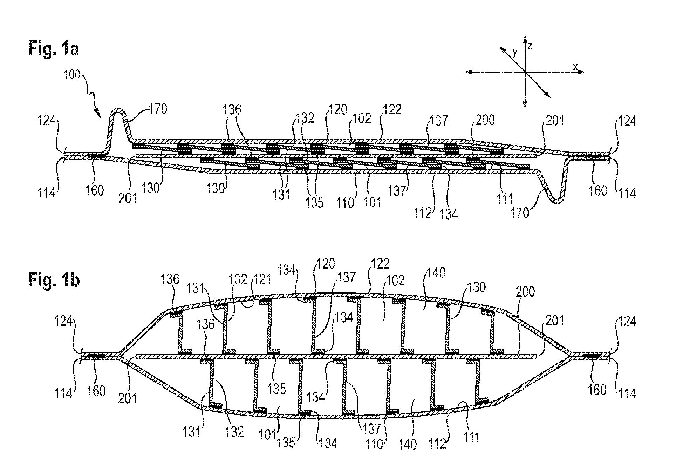

Figure 1A shows a multilevel cell-forming structure - with ligaments having Z-like shape- in its flat configuration whereas Figure 1B shows the structure in its erected configuration. The multilevel structure illustrated in Figs. 1A and 1B has two levels. The first level (101) is confined in a direction perpendicular to the longitudinal and lateral dimension by the first outermost layer (110) forming the subjacent layer of this level and by the first intermediate layer (200) (in this two-level structure there is however only one intermediate layer) forming the superjacent layer of this level. The second level (102) is confined in a direction perpendicular to the longitudinal and lateral dimension by the second outermost layer (120) forming the superjacent layer of this level and by the first intermediate layer (200) forming the subjacent layer of this level. Generally, for a multilevel structure, any given intermediate layer (200) forms the subjacent layer of one of the levels and also forms the superjacent layer of another level.

Generally, the structure (100) of the present invention comprises a first and a second outermost layer (110, 120). Moreover, the structure (100) comprises at least a first intermediate layer (200) and may comprise further intermediate layers, such as a second intermediate layer (210), third intermediate layer and additional intermediate layers. One or both of the first and second outermost layers (110, 120) and/or the intermediate layers (200, 210) may be non-elastic or highly non-elastic. Also one or both of the first and second outermost layers (110, 120) and/or the intermediate layers (200, 210) may be non-extensible or highly non-extensible. Given that elastic materials are often more expensive compared to non-elastic materials, it may be advantageous to use non-elastic, or highly non-elastic materials for the first and second outermost layer (110, 120) and/or the intermediate layers (200,210).

Moreover, if the first and second outermost layers are non-elastic, the overall structure may be more easily and reliably transferred from its initial flat configuration into its erected configuration, as the applied force is more readily used to erect the structure. If the first and second outermost layers are elastic, the applied forces may partly be converted into elongation of the first and second outermost layer alone, i.e. they are not used to erect the structure as a whole, depending on the elastic modulus of the elastic material. However, the use of elastic materials or highly elastic materials for the first and second outermost layer is also possible, especially if the elastic modulus is selected appropriately (typically, the elastic modulus should be relatively high). Similar considerations principally also apply to the use of extensible or highly extensible materials for the first and second outermost layer.

The first and second outermost layers and/or the intermediate layers may be made of nonwovens, film, paper, sheet-like foam, woven fabric, knitted fabric or combinations of these materials. Combinations of these materials may be laminates, e.g. a laminate of a film and a nonwoven. Generally, a laminate may consist of only two materials joined to each other in a face to face relationship and lying upon another but, alternatively may also comprise more than two materials joined to each other in a face to face and lying upon another.

The first and second outermost layer may be made of the same material. Alternatively, the first outermost layer may be made of material which is different from the material of the second outermost layer.

The first and second outermost layer may also be made of one continuous, single sheet of material which is folded over at one of the lateral edges of the structure. Alternatively, the first and second outermost layers may be made of separate sheets of material.

If the structure comprises more than one intermediate layer, the intermediate layers may be made of the same materials. The intermediate layers may also be made of the same material as the first and/or second outermost layer.

The materials of the first and second outermost layer may have the same basis weight, tensile strength, bending stiffness, liquid permeability, breathability and/or hydrophilicity. Alternatively, the materials of the first and second outermost layer may be chosen such that the first and second outermost layers differ from each other in one or more properties, such as basis weight, tensile strength, bending stiffness, liquid permeability, breathability and/or hydrophilicity. If the structure comprises more than one intermediate layer, the intermediate layers may have the same basis weight, tensile strength, bending stiffness, liquid permeability, breathability and/or hydrophilicity. Alternatively, the materials of the intermediate layers may be chosen such that the intermediate layers differ from each other and/or from the first and/or second outermost layer in one or more properties, such as basis weight, tensile strength, bending stiffness, liquid permeability, breathability and/or hydrophilicity.

The basis weight of the first outermost layer, the second outermost layer and the intermediate layer(s) may be at least 1 g/m$^2$, or at least 2 g/m$^2$, or at least 3 g/m$^2$, or at least 5 g/m$^2$; and the basis weight may further be not more than 1000 g/m$^2$, or not more than 500 g/m$^2$, or not more than 200 g/m$^2$, or not more than 100 g/m$^2$, or not more than 50 g/m$^2$, or not more than 30 g/m$^2$.

The tensile strength of the first and second outermost layers and of the intermediate layer(s) may be at least 3 N/cm, or at least 4 N/cm, or at least 5 N/cm. The tensile strength may be less than 100 N/cm, or less than 80 N/cm, or less than 50 N/cm, or less than 30 N/cm, or less than 20 N/cm.

The bending stiffness of the first and second outermost layer and the bending stiffness of the intermediate layer(s) may be at least 0.1 mNm, or at least 0.2 mNm, or at least 0.3 mNm. The bending stiffness may be less than 200 mNm, or less than 150 mNm, or less than 100 mNm, or less than 50 mNm, or less than 10 mNm, or less than 5 mNm.

Generally, the higher the tensile strength and the bending stiffness of the first and second outermost layer and of the intermediate layer(s), the more rigid, but also the more stable the overall structure will become. Hence, the choice of tensile strength and bending stiffness for the first and second outermost layer and for the intermediate layer(s) depends on the intended application of the structure, balancing overall softness, drape and conformability requirements with overall stability and robustness. In general, one may think the bending stiffness of the outermost layers and intermediate layers acting similar as a bridge and whatever is on top of the bridge bends it. The ligaments may be seen to act as columns of a bridge which technically decrease the free bending length of the bridge (however to the extent the ligament itself is capable of withstanding the pressure in vertical direction which, for ligaments having relatively low bending stiffness, may cause the ligament to buckle, i.e. bending out of plane under a force applied in the Z-direction of the structure). Sufficient bending stiffness of the outermost layers and the intermediate layers allows for transferring the load occurring due to an applied force in the Z-direction of the structure "downwards" through the structure. Outermost layers and intermediate layers having relatively high bending stiffness will be more readily able to withstand forces applied in the Z-direction, thus showing reduced bending.

The respective superjacent layer and subjacent layer of a level within the structure are connected to each other via ligaments (130).

Each of the first and second outermost layer (110, 120) and each intermediate layer (200, 210) in the structure (100) has a longitudinal dimension which is parallel to the longitudinal dimension of the structure (100) and which is confined by two spaced apart lateral edges (114, 124, 201, 211). Each of the first and second outermost layers (110, 120) and each intermediate layer (200, 210) also has a lateral dimension parallel with the lateral dimension of the structure and confined by two spaced apart longitudinal edges. The first and second outermost layer (110, 120) and the intermediate layer(s) (200, 210) in the structure (100) at least partly overlap each other.

Each level of the structure comprises one or more than one ligament (130). Each ligament (130) is provided between the superjacent layer and the subjacent layer of the respective level. Each ligament (130) has a longitudinal dimension confined by two spaced apart lateral edges (134) and a lateral dimension confined by two spaced apart longitudinal edges.

In Figure 1, a coordination system is shown with X-, Y- and Z-directions. The longitudinal dimension of the overall structure (100) and of the first and second outermost layer (110, 120) and the intermediate layer(s) (200, 210) extends along the longitudinal direction X of the coordination system. The longitudinal dimension of the ligaments (130) in the structure's initial flat configuration may substantially extend along the longitudinal direction X of the illustrated coordination system.

Likewise, the lateral dimension of the overall structure (100) and of the first and second outermost layer (110, 120) and the intermediate layer(s) (200, 210) extends along the lateral direction Y of the coordination system. The lateral dimension of the ligaments (130) in the structure's initial flat configuration may substantially extend along the lateral direction Y of the illustrated coordination system.

The caliper of the structure (100) extends along the Z direction of the coordination system.

Each ligament (130) is attached in a first ligament attachment region (135) at or adjacent to one of the ligament's lateral edges (134) to the subjacent layer of the respective level (i.e. the level by which the respective ligament is comprised). The ligament is attached to that surface of the subjacent layer which faces towards the superjacent layer of the respective level. Each ligament (130) is also attached in a second ligament attachment region (136) at or adjacent to other the ligament's lateral edges (134) to the superjacent layer of the respective level. The ligament is attached to that surface of the superjacent layer which faces towards the subjacent layer of the respective level. Due to this attachment, the ligaments (130) will generally adopt a C-like, Z-like, T-like, or Double-T-like shape when the structure is in its erected configuration.

Each ligament has a first surface (131) and a second surface (132). When the ligament adopts a Z-like shape when the structure is in its erected configuration, the ligament (130) is attached to the subjacent layer in the first ligament attachment region (135) with the ligament's first surface (131) at or adjacent to one of its lateral ligament edges (134) and is further attached to the superjacent layer in the second ligament attachment region (136) with the ligament's second surface (132) at or adjacent to its other lateral ligament edge (134).

Moreover, for Z-like shapes (shown, e.g. in Figs. 1A and 1B), the ligament's (130) first surface (131) may not be facing towards the superjacent layer when the structure (100) is in its initial flat configuration, and the ligament's second surface (132) may not be facing towards the subjacent layer when the structure (100) is in its initial flat configuration. Thereby, it is possible to obtain structures with no folds and hinges (or very few folds and hinges, e.g. when the ligaments have different longitudinal dimension in their free intermediate portion (137), see below). Hence, these structures will generally exhibit a lower tendency to partly erect in the absence of an applied force along the longitudinal dimension and will consequently remain in their initial flat configuration. Also, such structures will generally exhibit a greater tendency to return to their initial flat configuration when the applied force is released.

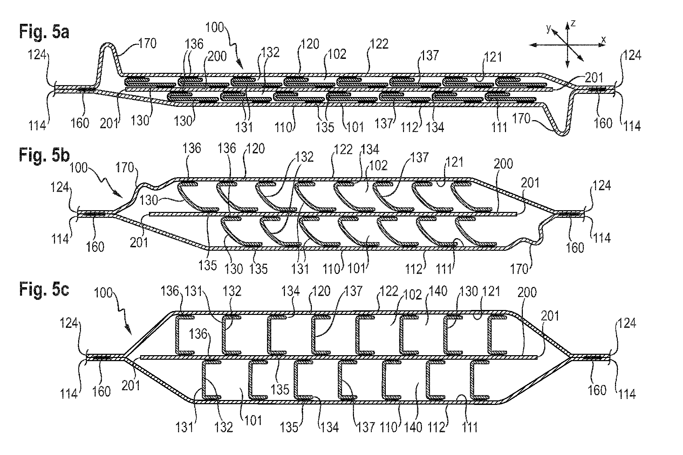

Alternatively, when the ligament adopts a C-like shape when the structure is in its erected configuration (exemplified in Figs. 5A to 5C), the ligament (130) is attached to the subjacent layer in the first ligament attachment region (135) with the ligament's first surface (131) at or adjacent to one of its lateral ligament edges (134) and is further attached to the superjacent layer in the second ligament attachment region (136) with the ligament's first surface (131) at or adjacent to its other lateral ligament edge (134).

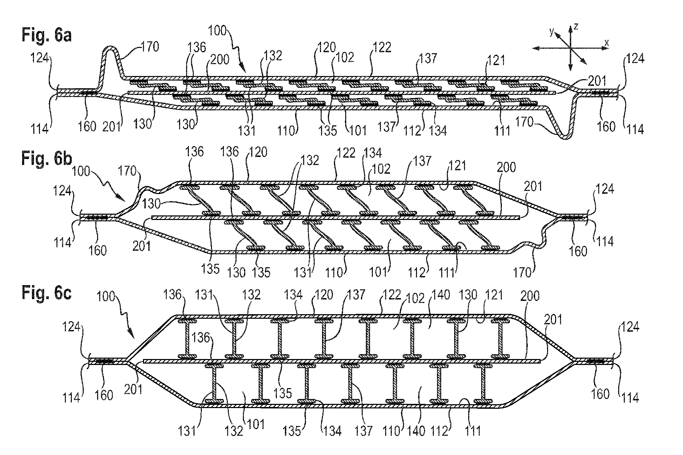

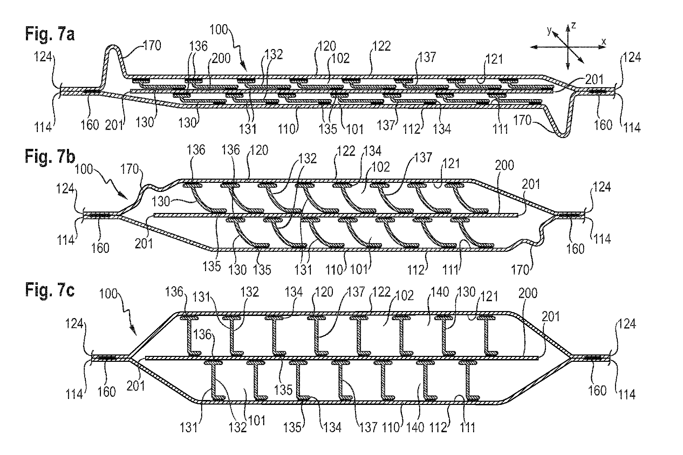

Still alternatively, one portion of the ligament at or adjacent to one of its lateral ligament edges may be attached to the subjacent layer such as to form a T-like (exemplified in Figs. 7A to 7C), or Double-T-like shape (exemplified in Figs. 6A to 6C). To facilitate such attachment, the portion of the ligament adjacent to a first lateral ligament edge has to comprise at least two ligament layers and the two ligament layers are not attached to each other in the portion of the ligament which is attached to the first layer. Thereby, the ligament can be split up and unfolded such that both ligament layers can be respectively attached to the subjacent layer in the first ligament attachment region (135) to form a T-like shape when the structure is in its erected configuration.

For embodiments where a ligament adopts a T-like shape, the portion of the ligament at or adjacent to the second lateral ligament edge is attached to the superjacent layer with either its first or second surface to form the second ligament attachment region (136). If the ligament is also made of a laminate in the second ligament attachment region (136), the ligament layers are not split up in this area (i.e. only the portion adjacent to the first lateral ligament edge forms a T-like shape).

When the ligament adopts a Double-T-like shape when the structure is in its erected configuration, the portion of the ligament at or adjacent to the second lateral ligament edge is attached to the superjacent layer similar to the manner in which the portion at or adjacent to the first lateral ligament edge is attached to the subjacent layer, i.e. the ligament laminate is split up and unfolded such that two ligament layers can be respectively attached to the superjacent layer to form the second ligament attachment region (136).

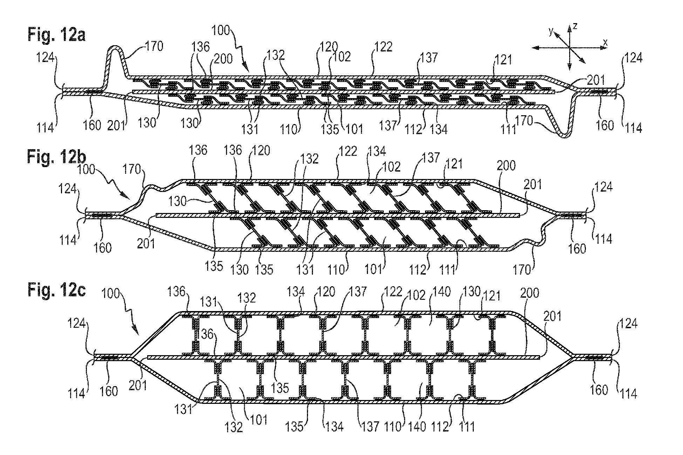

Splitting up and unfolding the ligament layers to form the respective first and second ligament attachment regions (135, 136) is exemplified in Figures 12A through 12C (showing ligaments with Double-T-like shape)

In a given structure, ligaments adopting a Z-like shape when the structure is in its erected configuration may all adopt the same orientation (see e.g. Figs. 1A and 1B). That means, in such embodiments none of the ligaments adopting a Z-like shape has an inverse configuration of another ligament adopting a Z-like shape.

Likewise, in a given structure, ligaments adopting a C-like shape when the structure is in its erected configuration may all adopt the same orientation (see e.g. Figs. 5A to 5C). That means, in such embodiments none of the ligaments adopting a C-like shape has an inverse configuration of another ligament adopting a C-like shape.

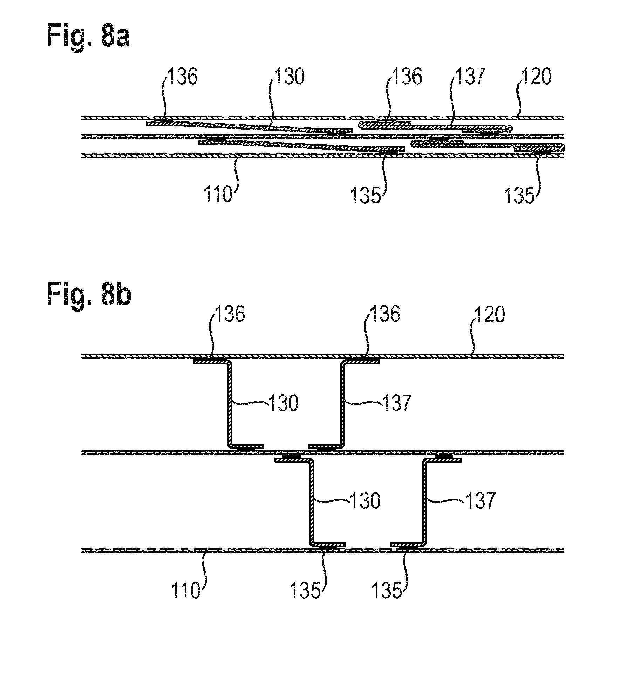

However, it is also possible to facilitate the structure such that that one or more ligaments adopt a Z-like shape which has an inverse configuration of one or more other ligaments with Z-like shape. An example of such structure is illustrated in Fig. 8A (flat configuration) and Fig. 8B (erected configuration).

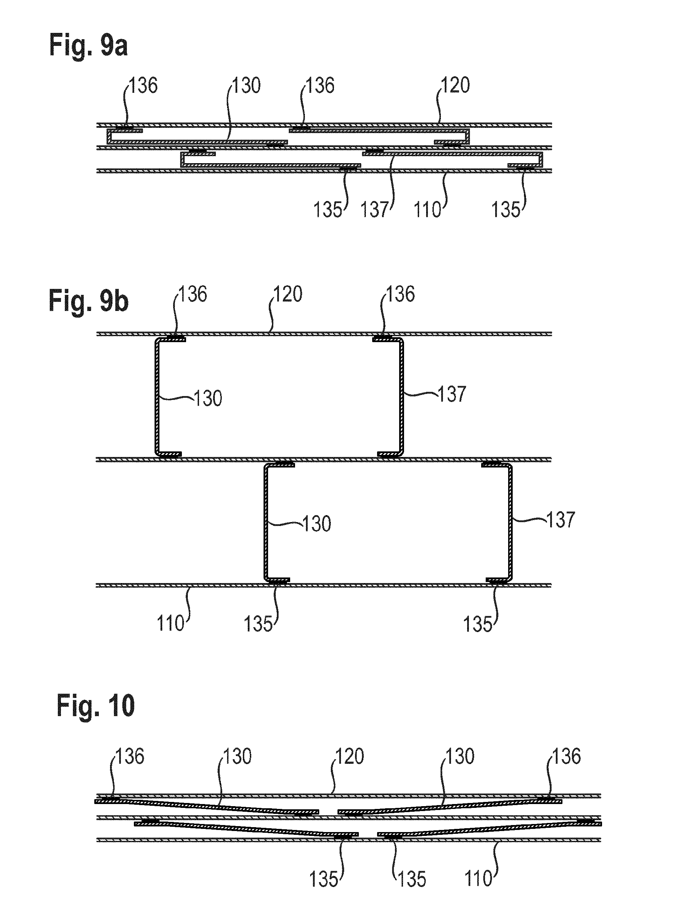

Similarly, a structure with one or more ligaments adopting a C-like shape which has an inverse configuration of one or more other ligaments with C-like shape is exemplified in Fig. 9A (flat configuration) and Fig.9B (erected configuration).

Such inverse configurations are feasible as long as the structure can be readily converted from its flat configuration to its erected configuration. An example of a structure having Z-like ligaments with inverse configuration which cannot be converted from its flat to erected configuration is shown in Fig. 10. In this structure, the ligaments on the right side of the Figure "block" the ligaments on the left side of the Figure from being erected upon application of a force along the longitudinal direction (and vice versa, the ligaments on the left side of the Figure "block" the ligaments on the left side of the Figure from being erected). The ligaments shown on the right side (or left side) of the Figure also cannot serve as a stop aid (explained below in detail) as no leeway is provided.

Generally, ligaments in a given structure have to be configured and attached accordingly such that the structure is able to be converted from an initial flat configuration into an erected configuration whereby the ligaments convert from an initial flat configuration into an erected configuration. Moreover, the ligaments in a given structure have to be configured and attached accordingly such that, upon further application of a force along the longitudinal dimension, it would be possible -in the absence of a means that maintains the structure in its erected configuration, such as a stop aid, which is described below- to convert the erected structure into a turned-over flat structure, wherein the ligaments would have been turned over by 180° based on the ligament's position in the initial flat structure configuration.

The longitudinal dimension of each ligament (130) between the first and second ligament attachment regions (135, 136) remains unattached to the subjacent and superjacent layer or is releasable attached to the subjacent and/or superjacent layer and/or to their neighboring ligament(s). This unattached or releasable attached portion is referred to as the "free intermediate portion" (137) of the ligament (130). "Releasable attached" means a temporary attachment to the subjacent and/or superjacent layer and/or to the neighboring ligament(s) in a way, that the bond strength is sufficiently weak to allow easy detachment from the subjacent and/or superjacent layer and/or the neighboring ligament(s) upon initial elongation of the structure (100) along the longitudinal dimension without rupturing or otherwise substantially damaging the ligaments (130) and/or the subjacent and/or superjacent layer and without substantially hindering the conversion of the structure from its initial flat configuration into its erected configuration.

When the structure is in its initial flat configuration, the first surface (131) of a ligament's free intermediate portion (137) faces towards the subjacent layer (110) and the second surface (132) of a ligament's free intermediate portion (137) faces towards the superjacent layer. When the structure (100) is converted into its erected configuration, the first surface (131) of a ligament's (130) free intermediate portion (137) faces towards the second surface (132) of its neighboring ligament (130). This is irrespective as to whether the ligament has been attached to adopt a Z-like, C-like, T-like or Double-T-like shape. Unless expressly mentioned herein, neighboring ligaments refers to ligaments which are neighboring along the longitudinal dimension.

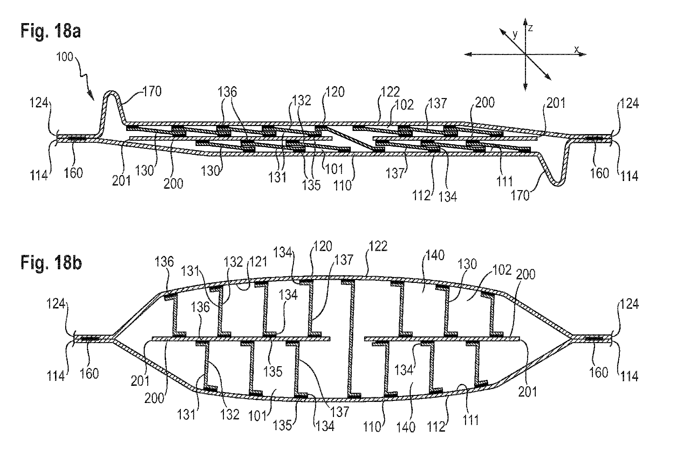

The structure may comprise one or more additional ligaments extending over more than one level across the caliper of the structure. For such ligaments, the ligament may be attached at or adjacent to one of its lateral ligament edges to the subjacent layer in a first ligament attachment area and the ligament may be further attached at or adjacent to its other lateral ligament edges to a layer above the subjacent layer in a second ligament attachment area. Such ligaments may be present if at least one intermediate layer is discontinuous across the longitudinal structure dimension and/or if at least one intermediate layer is not directly attached to another intermediate layer which neighbors the respective intermediate layer in the caliper direction (i.e. the neighboring intermediate layers are not attached to each other in the areas longitudinally outboard of the areas where the ligaments of the respective levels are provided). An example of such ligament is illustrated in Figs. 18A and 18B. Generally, for such ligaments extending over more than one level across the caliper of the structure, the same conditions and considerations apply with regard to the configuration of the attachment and ligament shape, as is set out above for the ligaments being comprised by only one level of the structure.

The ligaments (130) may be attached in their first and second ligament attachment region by any means known in the art, such as by use of adhesive, by thermal bonding, by mechanical bonding (such as pressure bonding), by ultrasonic bonding, or by combinations thereof. The attachment of the ligaments to the subjacent and superjacent layer is permanent, i.e. the attachment should not be releasable by forces which can typically be expected during use of the structure.

One, more than one or all of the intermediate layer(s) (200, 210) may not be attached to the first and/or second outermost layer (110, 120) other than indirectly via one or more ligaments and optionally indirectly via one or more stop aids (described below). Examples are shown in Figs. 1A and 1B.

However, one, more than one or all of the intermediate layers may be attached to the first and/or second outermost layer (110, 120) further to an indirect attachment via one or more ligaments and optionally indirectly via one or more stop aids, such as by direct attachment of this/these intermediate layer(s) to the first and/or second outermost layer (110, 120). One, more than one or all of the intermediate layer(s) (200, 210) may also be directly attached to each other.

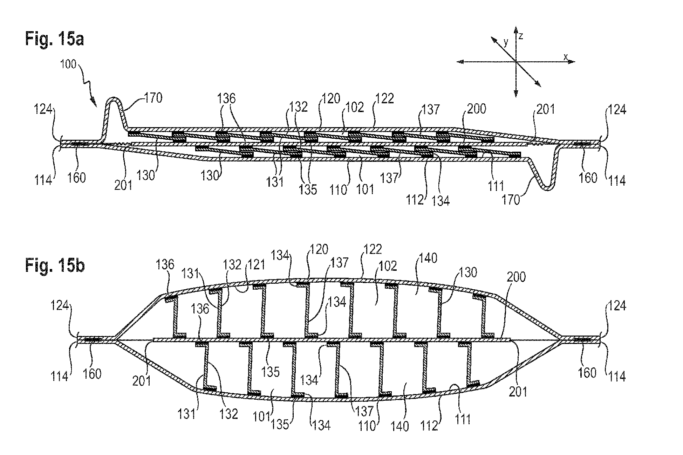

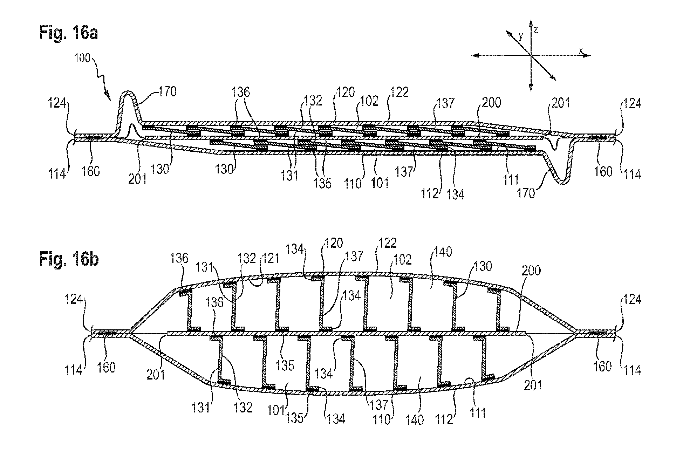

If provided, the attachment of the intermediate layer(s) to the first and/or second outermost layer (110, 120) and/or to each other may will be in the areas which are longitudinally outboard of the region where the ligaments (130) are provided, towards at least one of the lateral edges (114, 124) of the intermediate layer(s) (200, 210). (Direct) attachment can also be provided in two locations, i.e. towards both of the lateral edges of the intermediate layer(s) (200, 210), see e.g. Figs. 15A/15B and 16A/16B.

If one or more intermediate layer(s) (200, 210) are (directly) attached to the first and/or second outermost layer (110, 120) towards one or both of the lateral edges of the intermediate layer, the attachment must be such that conversion of the structure from its initial flat configuration into its erected configuration is not prevented or hindered prior to the desired degree of structure erection (as is set out below, it may be desirable to stop further elongation and erection of the structure before the structure has reached the maximum caliper which would be possible due to the longitudinal dimension of the ligaments).

Hence, the attachment is provided such that the intermediate layer which is attached to the first and/or second outermost layer (110, 120) has at least one predefined leeway, which may be between two neighboring ligaments which are attached to the respective intermediate layer, or may alternatively or in addition be between the attachment of the intermediate layer to the first and/or second outermost layer (110, 120) and the ligament which is attached to the respective intermediate layer closest to the attachment of the intermediate layer to the first and/or second outermost layer (110, 120). It is also possible to provide more than one predefined leeways. A leeway can form kind of a slack when the structure (100) is in its initial flat configuration, i.e. the longitudinal dimension of the intermediate layer in the leeway is larger than the longitudinal dimension of the structure in the area where the leeway is provided. An example of such leeway is shown in Figs. 16A and 16B. Alternatively, the leeway can be generated by adapting the material of the intermediate layer to create extensibility of the intermediate layer in this area, as is exemplified in Figs. 15A and 15B, wherein the extensibility of the material is schematically indicated by zigzags in the layer). Adapting the material can be done by modifying the material e.g. by selfing (weakening the material of the intermediate layer in the leeway to render it more easily extensible), or creating holes to form the leeway. Alternatively, the intermediate layer may be made of different material in the area of the leeway, with the material in the leeway being extensible and being more extensible than the remaining intermediate layer.

It is also possible to provide a leeway that is a combination of a slack and the provision of extensible material in the leeway, such that, upon elongation, initially the slack straightens out and subsequently, the extensible material elongates.

When a force is applied along the longitudinal dimension of the structure (100) to extend the structure, the first and second outermost layers (110, 120) shift against each other in opposite longitudinal directions, the ligaments are erected and the caliper of the structure (100) increases while the length of the structure increases simultaneously. When the first and second outermost layers (110, 120) shift against each other such that the leeway in the intermediate layer in form of a slack (170), which has been present in the initial flat configuration of the structure, flattens and straightens out (see Fig. 16B). Hence, elongation and erection of the structure is not prevented or hindered.

If the leeway is formed by creation of extensibility in respective area of the intermediate layer as described above, the intermediate layer elongates when the first and second outermost layers (110, 120) are shifted against each other until elongation is not possible any longer (without applying an excessive amount of force, which may even rupture the structure), see e.g. Fig. 15B. Hence, the material in the leeway has reached its maximum elongation, i.e. it cannot be elongated further upon application of force without causing damage to the structure that limits or impedes its intended use.

The material of the intermediate layer in the leeway may also be elastic. For such structures, the intermediate layer, in the area of the leeway, can retract when the force is no longer applied onto the structure such that the structure can substantially "snap back" into its initial flat configuration.

Attachment of the intermediate layer(s) to the first and second outermost layer (110, 120) and, if desired, to each other can be obtained by any means known in the art, such as adhesive, thermal bonding, mechanical bonding (e.g. pressure bonding), ultrasonic bonding, or combinations thereof. The attachment may be permanent, i.e. the attachment should not be releasable by forces which can typically be expected during use of the structure.

Alternatively, the attachment may be releasable. "Releasable attached" means a temporary attachment in a way, that the bond strength is sufficiently weak to allow easy detachment upon initial elongation of the structure (100) along the longitudinal dimension without rupturing or otherwise substantially damaging the structure and without substantially hindering the conversion of the structure from its initial flat configuration into its erected configuration.

One or more intermediate layers may also be discontinuous across the longitudinal dimension of the intermediate layer, such that the one or more intermediate layers comprise at least a first and a second section. An example of such a structure is shown in Figs. 18A and 18B, wherein the first intermediate layer (200) has a first and a second sub-section. In structures with one or more discontinuous intermediate layers, at least one ligament will be attached to each of the intermediate layer portions with one of its first or second ligament attachment regions.

Structures (100) as described supra are able to adopt an initial flat configuration when no external forces are applied. Upon application of a force along the longitudinal dimension, the structure will not only increase its longitudinal dimension, i.e. get longer, but simultaneously, the structure will also increase in caliper, i.e. in the direction perpendicular to the longitudinal and lateral dimension. Moreover, such structures typically do not exhibit necking upon elongation, i.e. the lateral dimension does not decrease.

Such structures may also return to essentially their initial longitudinal dimension and (flat) caliper upon release of the external force applied along the longitudinal dimension.

The force along the longitudinal dimension may be applied e.g. by grabbing the structure adjacent to the lateral edges (114, 124) of the first and second outermost layer (110, 120) (outside the area, where the ligaments (130) are positioned). The force may also be applied indirectly, i.e. without grabbing the structure, when the structure is built into an absorbent article, such as a disposable diaper or pant.

The structure can be facilitated such that, in its initial flat configuration, no hinges and folds may be created in the structure (100) and the first and second outermost layers (110, 120), the intermediate layer(s) as well as the ligaments (130) lie flat and outstretched (however, not extended beyond their dimension in the relaxed state). This is shown e.g. in Figs. 1A and 1B. Such structures allow having a very thin caliper in the flat configuration. In these kinds of structures, all ligaments will adopt a Z-like shape when the structure is in its erected configuration. Moreover, due to the absence of any folds and hinges in the initial flat configuration such structures will not exhibit any tendency to turn into a (partly) erected configuration when they are in the initial flat configuration without the application of an external force. Thus, they will more readily remain in a complete initial flat configuration with no external forces applied compared to structures with folds and hinges, which may exhibit some tendency to partly erect on their own motion depending on the properties of the materials selected for the first and second outermost layer, for the intermediate layer(s) and especially depending on the properties of the materials selected for the ligaments (such as bending stiffness).

In structures where no hinges may be created, the complete first surface (131) of each ligament (130) (i.e. not only the free intermediate portion) does not face towards the superjacent layer when the structure (100) is in its initial flat configuration, and the complete second surface (132) of each ligament (130) (i.e. not only the free intermediate portion) does not face towards the subjacent layer when the structure is in its initial flat configuration. Such a structure is illustrated in Figs. 1A and 1B.

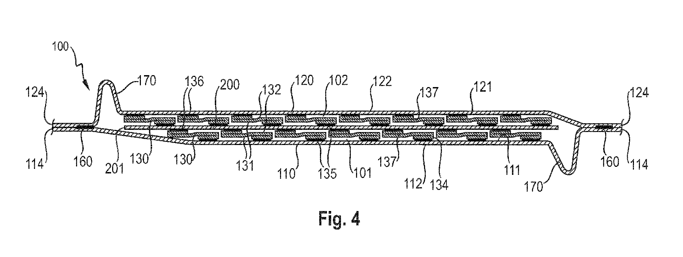

Fig. 4 shows a structure where the ligaments are folded when the structure is in its initial flat configuration.

Also, for certain other executions, e.g. those where the longitudinal dimension of the free intermediate portion (137) of the ligaments (130) differs from each other, the initial flat configuration may have some folds and hinges.

Upon application of a force along the longitudinal dimension of the structure (100), the first and second outermost layer (110, 120) shift relative to each other in opposite longitudinal directions such that the structure length extends. At the same time, the structure (100) erects due to the erection of the ligaments (130). Between neighboring ligaments, a space, a so-called "cell" (140) is formed, which is confined by the subjacent and superjacent layer and the respective neighboring ligaments. When viewed from the side, along the lateral direction, the cells may take for example a rectangular shape, a trapezoid shape, a rhomboid shape, or the like.

For structures wherein, for all levels, the longitudinal dimension of the free intermediate portion (137) is the same for all ligaments within a level, the structure (100) will adopt its highest possible caliper when the ligaments (130) are in an upright position, i.e. when the free intermediate portion (137) of the ligaments (130) is perpendicular to the first and second outermost layer (110, 120). However, the formation of this upright position may possibly be hindered, at least in some areas, when a force in a direction towards the caliper of the structure is applied at the same time, if this force is sufficiently high to deform the structure in the caliper dimension.

In structures, where the longitudinal dimension of the free intermediate portion (137) differs between different ligaments (130) within a level, the ligaments (130) may not be perpendicular to the first and second outermost layer (110, 120) in the erected configuration. In such embodiments, the first and second outermost layer (110, 120) are not parallel to each other when the structure is in its erected configuration but instead, the first and/or second outermost layer (110, 120) take(s) an inclined shape.

Tensile strength, and especially bending stiffness, impacts the resistance of the structure (especially of the structure in its erected configuration) against compression forces. Thus, when the ligaments have a relatively high tensile strength and bending stiffness, the structure is more resistant to forces applied in the Z-direction (i.e. towards the caliper of the structure). Also, if the first and/or second layers have relatively high tensile strength and relatively high bending stiffness, resistance of the structure against forces applied in the Z-direction (i.e. towards the caliper of the structure) is increased. For the present invention, the compression resistance of the erected structure is measured in terms of the structure's modulus according to the test method set out below.

As a difference in bending stiffness results in a difference in the resistance against compression forces (and hence, the modulus) applied in the Z-direction (i.e. towards the caliper of the structure), using ligaments with different bending stiffness enables structures which have improved resistance to compression (higher modulus) in the Z-direction in areas where the ligaments have higher bending stiffness, whereas the structure adjusts more readily e.g. to curved surfaces in the areas where ligaments with lower bending stiffness are applied (lower structure modulus). For example, the bending stiffness of the ligaments which are arranged in the center of the structure along the longitudinal dimension may be higher compared to the ligaments arranged towards the lateral edges of the structure.

Not only the bending stiffness and/or tensile strength of different ligaments comprised by the same level may differ from each other, but in addition to or instead of having ligaments with varying bending stiffness and/or tensile strength within the same level, ligaments comprised by one or more levels may also differ in bending stiffness and/or tensile strength from ligaments comprised by one or more other levels.

When the structure is comprised by an absorbent article, the ligaments of the level which is closest to the skin of the wearer when the article is in use, may have a lower bending stiffness and/or lower tensile strength compared to ligaments of levels being farther from the skin of the wearer.

When the ligaments of a level differ from each other in bending stiffness and/or tensile strength, one or more ligaments which are closer to the lateral edges of the multi-level structure may have a lower bending stiffness and/or lower tensile strength compared to one or more ligaments which are arranged in or towards the center of the multi-level structure along the longitudinal dimension.

In addition to the tensile strength and the bending stiffness of the materials used for the structure, the resistance of the (erected) structure against compression forces is also impacted by the number of ligaments which are provided, and the distance between neighboring ligaments. Neighboring ligaments which have a relatively small gap between them along the longitudinal dimension of the structure will provide for higher resistance of the erected structure against compression forces (higher modulus) compared to a structure wherein neighboring ligaments are more widely spaced apart along the longitudinal dimension of the structure (lower modulus) as long as the material used for the different ligaments and their size does not differ from each other.

Different levels may have different numbers of ligaments. In addition to or instead of having different number of ligaments in different levels, the ligaments may also be spaced at different distances from each other. Generally, the distance between neighboring ligaments may be constant or may vary within a given level.

Furthermore, the ligaments of different levels may be arranged such that their position coincides in the thickness direction of the structure (i.e. perpendicular to the longitudinal and lateral structure dimension). Alternatively, the ligaments of different levels may be staggered such that their position does not coincide in the thickness direction of the structure. Combinations of the aforementioned are also possible, i.e. the position of ligaments between two or more levels coincides while the position of ligaments between these two or more levels and one or more other levels does not coincide. In a still further alternative, the position of some ligaments within a level may coincide with the position of some or all (if the other level has fewer ligaments) ligaments of one or more other levels, while the position of other ligaments within this level does not coincide with the position of one or more ligaments of another level. Generally, a multitude of arrangements, positions and/or distributions of ligaments in the different levels are feasible.

Moreover, if the modulus is measured between neighboring ligaments, the modulus will typically be lower than the modulus measured in the location where a ligament is positioned. Modulus is measured following the test method set out below and is measured in the Z-direction of the structure.

The structure in its erected configuration may have a modulus of at least 0.004 N/mm$^2$, or at least 0.01 N/mm$^2$, or at least 0.02 N/mm$^2$, or at least 0.03 N/mm$^2$ in those areas where a ligament is posited as well as in the areas between neighboring ligaments.

Moreover, for certain applications, it may also be desirable to avoid excessively high compression resistance (i.e. too high modulus), e.g. to avoid that the erected structure is too stiff. This may be preferred when certain conformity of the structure to a surface (such as skin) is desirable. For such structures, the structure in its erected configuration may have a modulus of not more than 1.0 N/mm$^2$, or not more than 0.5 N/mm$^2$, or not more than 0.2 N/mm$^2$, but at least

0.1 N, or at least 0.5 N, or at least 1.0 N in those areas where a ligament is posited as well as in the areas between neighboring ligaments.

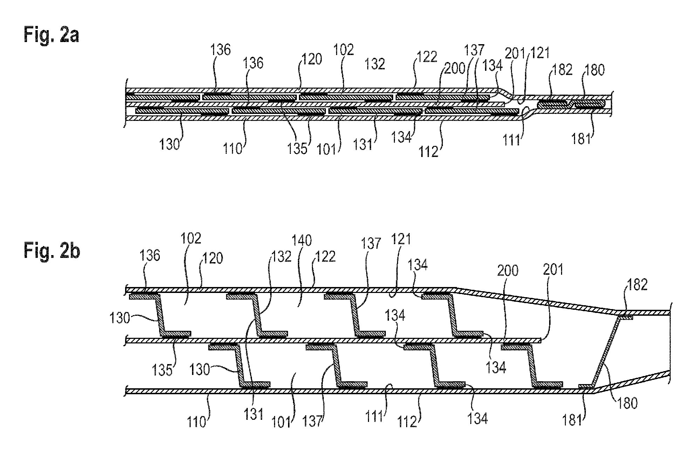

Generally, the ligaments (130) of a given level may be spaced apart from each other along the longitudinal dimension at equal distances or, alternatively, at varying distances (this may be applicable to each level or to only one or some levels). Also, the ligaments (130) of a given level may be spaced apart from each other such, that the ligaments (130) of given level do not overlap with each other when the structure (100) is in its initial flat configuration (again, this may be applicable to each level or to only one or some levels). Thereby, it is possible to provide structures (100) with very small caliper when the structure (100) is in its initial flat configuration, as the ligaments (130) do not "pile up" one on top of each other when the structure is in its initial flat configuration. An example of such structure is shown in Figs. 2A and 2B, wherein the ligaments do not overlap with each other within both, the first and the second level.

The free intermediate portion (137) of the ligaments (130) within each level may all have the same longitudinal dimension (the free intermediate portion (137) of the ligaments between different levels may have different longitudinal dimensions). Thereby, the structure will have a constant caliper across its longitudinal (and lateral) dimension when the structure (100) is in its erected configuration (except for the areas longitudinally outward of the regions where the ligaments are placed, towards the lateral edges of the structure). An example of such an embodiment is shown in Figure 1B. Alternatively, the longitudinal dimension of the free intermediate portion (137) may vary for different ligaments (130) within a level of a structure (100). Thereby, the caliper of the structure will vary across the longitudinal dimension. The free intermediate portion (137) of neighboring ligaments (130) may increase or decrease along the longitudinal dimension of the structure, or the free intermediate portion (137) may vary randomly along the longitudinal dimension, depending on the desired shape of the structure in its erected configuration and on the intended use of the structure. Also, the longitudinal dimension of the free intermediate portion (137) of the ligaments (130) may be the same within one or some levels while it varies for the ligaments within one or more other levels.

For example, the one or more ligaments (130) in the center of the structure (as seen along the longitudinal dimension) of one or more levels (may also be all levels) may have a longer free intermediate portion (137) compared to the ligaments towards the lateral edges of the structure, resulting in a structure with a overall higher caliper in the center than towards the edges when the structure is in its erected configuration. Thereby, the resulting erected structure may, for example, adopt a rhomboid or trapeze shape (when viewed from the side) Also, one or more ligaments (130) towards one of the lateral edges of one or more levels (may also be all levels) may have a longer free intermediate portion (137) than one or more ligaments towards the other lateral edge, resulting in a structure with a wedge-like shape (when viewed from the side) when the structure is in its erected configuration. Generally, the caliper and shape of the erected structure will depend on the length of the free intermediate portion (137) of the ligaments (130).

Generally, the maximum increase in caliper of the structure in its erected configuration (versus the caliper of the structure in its initial flat configuration) depends mainly on the longitudinal dimension of the free intermediate portion (137) of the ligaments (130). Given that the structure has one or more levels (such as two, three, four or even more levels), the overall caliper depends on the longitudinal dimension of the ligaments in all levels.

If a stop aid (160, 180, 190) is used (as described below), the structure may not be able to adopt its maximum increase in caliper in its erected configuration as the erection is stopped by the stop aid before the maximum erection, which would have been possible in the absence of a stop aid, is reached.

The maximum caliper of the erected structure versus the structure in its initial flat configuration is measured according to the test method set out below and may be at least 2 mm, or at least 3 mm, or at least 4 mm, or at least 5 mm, or at least 7 mm, or at least 10 mm, and may be less than 120 mm, or less than 100 mm, or less than 70 mm, or less than 50 mm, or less than 40 mm, or less than 30 mm, or less than 25 mm, or less than 20 mm, or less than 15 mm, or less than 10 mm, or less than 5 mm.

If formation of wrinkles in the first and second outermost layer (110, 120), in the intermediate layer(s) (200, 210) and/or in the ligament (130) shall be avoided when the structure is in its erected configuration, it is desirable, that the ligaments (130) are arranged such that, when the structure is in its initial flat configuration, the longitudinal dimension of the ligaments is substantially parallel with the longitudinal dimension of the first and second outermost layer and with the intermediate layer(s). "Substantially parallel" means that the orientation of the longitudinal dimension of the ligaments does not deviate by more than 20°, or not more than 10°, or not more than 5°, or not more than 2° from the longitudinal dimensions of the first and second outermost layer and the intermediate layer(s). The orientation of the longitudinal dimension of the ligaments may also not deviate at all from the longitudinal dimension of the first and second outermost layer and of the intermediate layer(s).

Typically, the free intermediate portions (137) are not attached to each other.

Depending on the materials used for the structure, especially depending on the material used for the ligaments (130) and the manner in which the ligaments are attached to the subjacent and superjacent layer, the erected structure may or may not return substantially completely to its initial flat configuration upon release of the force applied along the longitudinal dimension, as can be determined when a structure has been erected to adopt substantially its maximum possible caliper and has been held in this position for 5 minutes and immediately after it is allowed to relax for 1 minute upon release of the force applied along the longitudinal direction.

Generally, the first and second outermost layer (110, 120) may have the same lateral dimension and the longitudinal edges of the first and second outermost layer may be congruent with each other. The intermediate layer(s) (200, 210) may also have the same lateral dimension and the longitudinal edges of the intermediate layer(s) may be congruent with each other (if more than one intermediate layer is used) and may also be congruent with the first and second outermost layers.

Also, the lateral dimension of all ligaments (130) may be the same, and the lateral dimension of all ligaments (130) may also be the same as the lateral dimension of the respective subjacent and superjacent layers. The longitudinal edge of the first outermost layer (110) may coincide with the longitudinal edge of the second outermost layer (120), may further coincide with the longitudinal edge of the intermediate layer(s) and/or with the longitudinal edges of the ligaments (130).

However, the intermediate layer(s) (200, 210) may also have a smaller or wider longitudinal dimension as the first and second outermost layer, such that the structure has narrower or wider portions in along its caliper.

Also, one or more of the ligaments (130) may have the same lateral dimension as the respective subjacent and superjacent layers and one or more of the ligaments, which do not have the same lateral dimension as the respective subjacent and superjacent layer may be flanked on one or both longitudinal edges by other ligaments, such that the structure has laterally neighboring ligaments.

Generally, the structure may have a longitudinal and/or lateral dimension of at least 4 cm, or at least 5 cm, or at least 6 cm, or at least 7 cm and may have a longitudinal and/or lateral dimension of not more than 100 cm, or not more than 50 cm, or not more than 30 cm, or not more than 20 cm. If the longitudinal dimension is not the same along the lateral direction, the minimum longitudinal dimension is determined at the location where the longitudinal dimension has its minimum and the maximum longitudinal dimension is determined at the location where the longitudinal dimension has its maximum. Similarly, if the lateral dimension is not the same along the longitudinal direction, the maximum lateral dimension is determined at the location where the lateral dimension has its maximum and the minimum lateral dimension is determined at the location where the lateral dimension has its minimum. The structure may have an overall rectangular shape.

The longitudinal and lateral dimensions are determined when the structure is in its initial flat configuration.

The free intermediate portion (137) of the ligaments (130) may have a longitudinal dimension of at least 2 mm, or at least 3 mm, or at least 4 mm, or at least 5 mm, or at least 7 mm, or at least 10 mm, and may have a longitudinal dimension of less than 100 mm, or less than 70 mm, or less than 50 mm, or less than 40 mm, or less than 30 mm, or less than 25 mm, or less than 20 mm, or less than 15 mm, or less than 10 mm, or less than 5 mm.

Ligaments

The ligaments may be elastic, non-elastic or highly non-elastic. Also, the ligaments may be extensible, non-extensible or highly non-extensible. The ligaments may be made of nonwovens, film, paper, or sheet-like foam, woven fabric, knitted fabric, or combinations of these materials. Combinations of these materials may be laminates, e.g. a laminate of a film and a nonwoven.

The ligaments within a structure may all be made of the same material or, alternatively, the different ligaments within a structure may be made of different materials.

The material may be the same throughout each ligament, i.e. the ligament may be made of a single piece of material or of a laminate wherein each laminate layer extends over the complete ligament.

The ligaments may have the same properties throughout the ligament, especially with regard to bending stiffness and tensile strength.

Alternatively, the ligaments may have areas with properties (such as bending stiffness and/or tensile strength) which differ from the properties in one or more other areas of a given ligament.

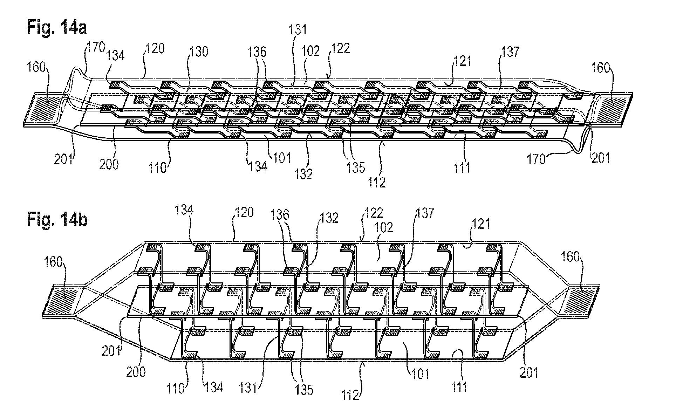

Such areas with differing properties can be facilitated by modifying the material in one or more ligament areas, e.g. by mechanical modification. Non-limiting examples of mechanical modifications are the provision of cut outs in one or more areas of the ligament to reduce tensile strength and bending stiffness in those areas; incremental stretching (so-called "ring-rolling") one or more ligament areas to reduce tensile strength and bending stiffness; slitting one or more ligament areas to reduce tensile strength and bending stiffness; applying pressure and/or heat to one or more areas of ligaments; or combinations of such mechanical modifications. Application of heat and/or pressure may either increase or reduce tensile strength and bending stiffness: For example, if heat and/or pressure are applied on a ligament made of nonwoven with fibers made of thermoplastic material, the fibers may be molten together and bending stiffness and tensile strength can be increased. However, if an excessive amount of heat and/or pressure is used, the material of the ligaments may be damaged (such as fiber breakage in a nonwoven web) and weakened areas are formed, thus reducing bending stiffness and tensile strength. Cutting out areas of the ligaments may either result in the formation of apertures within the ligament or the cut out may not be fully surrounded by uncut areas. An example is shown in Figs. 14A and 14B.

Alternatively, or in addition to the above, areas with different properties can also be obtained by chemically modifying one or more areas of the ligament, e.g. by adding chemical compounds, such as binders or thermoplastic compositions to increase bending stiffness and tensile strength, which may be followed by curing. One or more areas with different properties can also be obtained by providing different materials in different areas or by adding additional pieces of materials only in certain areas (thus, e.g. forming a laminate in these areas), while having another material (which may be the same or different from the piece of material added in certain areas) which is coextensive with the ligament and used throughout the ligament.

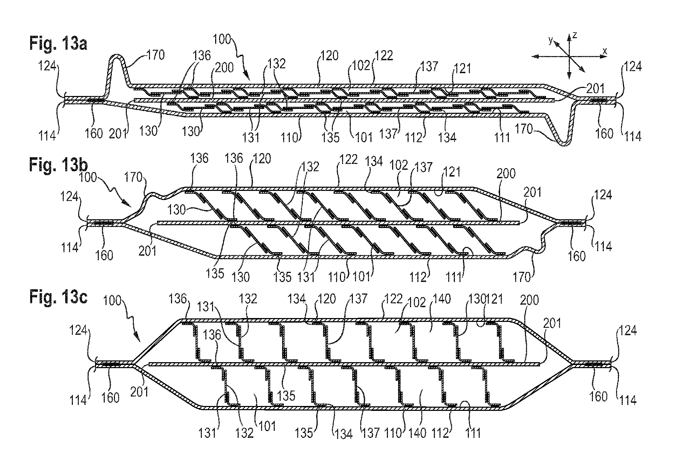

Still further, one or more areas with different properties may be achieved by providing ligaments which are assembled by attaching different pieces of material to each other so they overlap partly and partly do not overlap, such that together they form the overall ligament (instead of having one continuous material coextensive with the ligament to which additional pieces of material(s) are added only in certain areas). Examples of structures with ligaments being assembled of pieces of (different) materials are illustrated in Fig. 12A through 12C and 13A through 13C.

By having ligaments with one or more areas having properties different from the remaining ligament, the behavior of the structure with respect to e.g. bending stiffness and tensile strength can be fine tuned to meet certain needs in different areas of the structure (e.g. the ability to accommodate readily and softly to the skin of a wearer in some areas and to be stiffer and more resistant to compression in other areas to close gaps).

If providing such areas of different properties by any of the above means, it may be especially desirable to provide them in the areas of the free intermediate portion of a ligament which are directly adjacent to the first and second ligament attachment region. The areas of the free intermediate portion which are directly adjacent to the first and second ligament attachment regions are those areas which bend upon application of a force along the longitudinal direction of the structure, thus erecting the free intermediate portion.

For example, by having higher bending stiffness and/or tensile strength in the areas of the free intermediate portion directly adjacent to the first and second ligament attachment regions, results in ligaments which have a higher tendency to convert back from the erected configuration to the initial flat configuration upon relaxation of the force applied along the longitudinal dimension.

Alternatively, having lower bending stiffness and/or tensile strength in the areas of the free intermediate portion directly adjacent to the first and second ligament attachment regions, results in ligaments which have a lower tendency to convert back from the erected configuration to the initial flat configuration upon relaxation of the force applied along the longitudinal dimension (i.e. have a higher tendency to remain erected or at least partly erected). Such structures would also require less force to be converted into their erected configuration, as the ligament's free intermediate portion would bend more readily in the areas directly adjacent to the first and second ligament attachment regions.

The basis weight of each of the ligaments may be at least 1 $g/m^2$, or at least 2 $g/m^2$, or at least 3 $g/m^2$, or at least 5 $g/m^2$; and the basis weight may further be not more than 1000 $g/m^2$, or not more than 500 $g/m^2$, or not more than 200 $g/m^2$, or not more than 100 $g/m^2$, or not more than 50 $g/m^2$, or not more than 30 $g/m^2$.

If the tensile strength is the same throughout the ligament, the tensile strength of the ligaments may be at least 3 N/cm, or at least 5 N/cm or at least 10 N/cm. The tensile strength may be less than 100 N/cm, or less than 80 N/cm, or less than 70 N/cm, or less than 50 N/cm, or less than 40 N/cm.

If the tensile strength is the same throughout the ligament, the bending stiffness of the ligaments may be at least 0.1 mNm, or at least 0.2 mNm, or at least 0.3 mNm. The bending stiffness may be less than 500 mNm, or less than 300 mNm, or less than 200 mNm, or less than 150 mNm. Principally, for the ligaments the same considerations regarding overall softness, drape and conformability versus overall stability and robustness apply as set out above for the first and second layer. However, the bending stiffness and tensile strength of the ligaments typically has a higher impact on the overall bending resistance of the erected structure (when a force is applied along the caliper of the structure, i.e. perpendicular to the lateral and longitudinal dimension of the structure) vs. the impact of the bending stiffness and tensile strength of the first and second layer. Thus, it may be desirable that the ligaments have a higher bending stiffness and a higher tensile strength than the first and second layer.

The different ligaments in a structure may vary from each other in basis weigh and/or tensile strength, bending stiffness and the like.

## Stop aid

It may be desirable to define a maximum shifting of the first and second outermost layers (110, 120) relative to each other in opposite longitudinal directions upon application of the force along the longitudinal dimension of the structure (100). This can be facilitated by providing a stop aid (160; 180; 190).

By using a stop aid (160; 180; 190), the structure (100) is stopped in a defined erected configuration, i.e. with a defined caliper (which, however, is higher than the caliper of the structure in its flat configuration), even if the force along the longitudinal dimension is continued to be applied. The stop aid (160; 180; 190) may ensure that the structure (100) is stopped in the erected configuration with the highest caliper as enabled by the free intermediate portion (137) of the ligaments (130) while the force in the longitudinal dimension is continued to be applied. Alternatively, the stop aid (160; 180; 190) can also facilitate that the structure (100) is stopped in the erected configuration with a certain caliper, which is higher than the caliper of the initial flat configuration but lower than the highest possible caliper which would be possible due to the longitudinal dimension of the free intermediate portion (137) of the ligaments (130). Generally, the stop aid (160; 180; 190), when comprised by the structure (100), can avoid that the structure "over-expands" when a force in the longitudinal dimension is applied, such that the ligaments cannot transition from an initial flat configuration into an erected configuration and further onto a flattened configuration in which the ligaments are turned over by 180°.

There are many different ways to provide a stop aid (160; 180; 190), for example:

Moreover, upon complete flattening and straightening out of the leeway(s) in the intermediate layer the structure (100) cannot be extended any further upon application of a force in the longitudinal dimension. Hence, shifting is stopped and the structure has reached its "final" length and caliper in the erected configuration.

The leeway(s) provided in the intermediate layer thus act as a stop aid. Further ways to execute a stop aid are described in more detail below.

a) The first and second outermost layer (110, 120) are attached to each other in at least one layer-on-layer attachment region (160), which may for example be longitudinally outboard of the region where the ligaments (130) are provided, towards one of the lateral edges (114, 124) of the first and/or second outermost layer (110, 120). This layer-on-layer attachment region (160) is provided such that one of the first and second outermost layers (110, 120) has at least one predefined leeway, which may be between two neighboring ligaments, or may alternatively or in addition be between the layer-on-layer attachment region (160) and the ligament attachment region (135, 136) of that ligament which is closest to the layer-on-layer attachment region (160) and attached to the respective outermost layer where the leeway is provided. It is also possible to provide more than one predefined leeways which, in combination, define the maximum possible elongation of the structure. A leeway can form kind of a slack (170) when the structure (100) is in its initial flat configuration, i.e. the longitudinal dimension of the first and/or second outermost layer in the leeway is larger than the longitudinal dimension of the structure in the area where the leeway is provided. An example of such stop aid is shown e.g. in Figs. 1A, 1B, 4, 5A to 5C, 6A to 6C, 7A to 7C). Alternatively, the leeway can be generated by adapting the material of the first or second outermost layer (110, 120) in the area where the leeway is to be provided to create extensibility of the respective layer in this area. Adapting the material can be done by modifying the material e.g. by selfing (weakening the material of the respective first or second layer in the leeway to render it relatively easily extensible), creating holes or using extensible materials to form the leeway. Alternatively, the first or second layer outermost may be made of different material in the area of the leeway, with the material in the leeway being extensible.

It is also possible to provide a leeway that is a combination of a slack and the provision of extensible material in the leeway, such that, upon elongation, initially the slack straightens out and subsequently, the extensible material elongates.

When a force is applied along the longitudinal dimension of the structure (100) to extend the structure, the first and second outermost layers (110, 120) shift against each other in opposite longitudinal directions, the ligaments are erected and the caliper of the structure (100) increases while the length of the structure increases simultaneously. When the first and second outermost layers (110, 120) have been shifted against each other such that the leeway in form of a slack (170), which has been present in the initial flat configuration of the structure, has flattened and straightened out, the structure (100) cannot be extended any further upon application of a force in the longitudinal dimension. Hence, shifting is stopped and the structure has reached its "final" length and caliper in the erected configuration. If the leeway is formed by creation of extensibility in the first or second outermost layer as described above, the first or second outermost layer elongates when the first and second outermost layers (110, 120) are shifted against each other until elongation is not possible any longer (without applying an excessive amount of force, which may even rupture the structure). Hence, the material in the leeway has reached its maximum elongation i.e. it cannot be elongated further upon application of force without causing damage to the structure that limits or impedes its intended use.

Either only one layer-on-layer attachment region (160) can be provided, or, alternatively, two layer-on-layer attachment regions (160) can be provided, in each of the first and second outermost layer (110, 120). If two layer-on-layer attachment regions (160) are provided, one or more leeway(s) is/are provided in the first outermost layer (110), e.g. towards one of the lateral edges (135) and one or more other leeway(s) is/are provided in the second outermost layer (120), e.g. towards the respective other lateral edge (136) of the structure. Upon extending the structure (100) by applying a force along the longitudinal dimension, the leeways will flatten and straighten out, or if one or more leeways have been obtained by rendering the first or second outermost layer extensible in the respective area, these leeways will elongate until they have reached their maximum elongation.

The material of the layer-on-layer leeway may also be elastic. For such structures, the layer-on-layer stop aid (160) can retract when the force is no longer applied onto the structure such that the structure can substantially "snap back" into its initial flat configuration.

However, if the leeway is extensible (but non-elastic) or if the leeway is elastic, the properties of the leeway have to be such that the leeway does not elongate further when a certain elongation has been reached (i.e. when the structure has erected to the predetermined, desired extend). For many extensible and elastic materials, the materials elongate when a certain force is applied until a certain extension has been reached. Then, due to the material properties, a considerably higher force is needed (often referred to as "force wall"). Thereafter, upon further elongation, the material breaks and ruptures (as may be the case for any other materials when an excessively high force is applied). Selecting appropriate materials and properties for a given application of the structure will be based on the technical knowledge of persons familiar with such materials.

Attachment of the first and second outermost layer (110, 120) to each other in the one or more layer-on-layer attachment regions (160) can be obtained by any means known in the art, such as adhesive, thermal bonding, mechanical bonding (e.g. pressure bonding), ultrasonic bonding, or combinations thereof.

b) A layer-to-layer stop aid (180) may be provided, which extends from the first outermost layer (110) to the second outermost layer (120). This layer-to-layer stop aid (180) is attached to the inner surface (111) or outer surface (112) of the first outermost layer (110) in a first layer-to-layer stop aid attachment region (181) and is further attached to the inner surface (121) or outer surface (122) of the second outermost layer (120) in a second layer-to-layer stop aid attachment region (182). The first layer-to-layer stop aid attachment region (181) may be longitudinally spaced apart from the second layer-to-layer stop aid attachment region (182) when the structure is in its initial flat configuration. The layer-to-layer stop aid (180) is provided with a layer-to-layer stop aid leeway between the first and second layer-to-layer stop aid attachment regions (181, 182) when the structure (100) is in its initial flat configuration. The leeway may be configured in form of a slack (183). Upon application of a force along the longitudinal dimension the first and second layers (110, 120) shift relative to each other in opposite longitudinal directions, the structure extends and erects, and the slack (183) forming the layer-to-layer stop aid leeway between the first and second layer-to-layer stop aid attachment regions (181, 182) straightens out. Once the slack (183) is flattened when the structure (100) is in its erected position, further longitudinal extension of the structure is inhibited also when the force in the longitudinal dimension is continued to be applied. An example of a layer-to-layer stop aid (180) is illustrated in Figs. 2A (initial flat configuration) and 2B (erected configuration).

Alternatively or in addition, the leeway of the layer-to-layer stop aid (180) can be generated by adapting the material between the first and second layer-to-layer stop aid attachment regions (181, 182) to create extensibility of the respective area of the layer-to-layer stop aid (180). Adapting the material can be done by modifying the material, e.g. by selfing (weakening the material of the first or second layer to render it relatively easily extensible), creating holes or using extensible materials to form the leeway.

Alternatively or in addition, the layer-to-layer stop aid (180) may be made of extensible material. For such layer-to-layer stop aids (180), the material of the leeway elongates when the first and second outermost layers (110, 120) are shifted against each other until elongation of the layer-to-layer stop aid leeway is not possible any longer (without applying an excessive amount of force, which may even rupture the structure). Hence, the material in the leeway has reached its maximum elongation i.e. it cannot be elongated further upon application of force without causing damage to the structure that limits or impedes its intended use.

The material of the leeway may also be elastic. For such structures, the layer-to-layer stop aid (180) can retract when the force is no longer applied onto the structure such that the structure can substantially "snap back" into its initial flat configuration.

For the material properties and appropriate selection of extensible or elastic leeways, the same considerations apply as are set out above for the layer-on-layer stop aid.

It is also possible to provide a leeway that is a combination of a slack and the provision of extensible material in the leeway, such that, upon elongation, initially the slack straightens out and subsequently, the extensible material elongates.

Compared to the attachment of the ligaments to the first and second outermost layer and the resulting configuration, the layer-to-layer stop aid, when applied between the first and second outermost layer and being attached to the inner surfaces of the first and second layer, does not adopt a Z-like or C-like shape having the same orientation as the ligaments with Z-like or C-like shape (otherwise, it would simply be an additional ligament). Instead, the first layer-to-layer stop aid attachment region may be between the first surface of the layer-to-layer stop aid and the inner surface of the first outermost layer. The second layer-to-layer stop aid attachment region may be between the first surface of the layer-to-layer stop aid (i.e. on the same surface of the layer-to-layer stop aid as the first layer-to-layer stop aid attachment region) and the inner surface of the second outermost layer. Thus, the layer-to-layer stop aid adopts a C-like shape when the structure is in its erected configuration.

Alternatively or in addition, the first layer-to-layer stop aid attachment region may be between a first surface of the layer-to-layer stop aid and the inner surface of the first outermost layer The second layer-to-layer stop aid attachment region may be between the second surface of the layer-to-layer stop aid (i.e. opposite surface of the layer-to-layer stop aid than the first layer-to-layer stop aid attachment region) and the inner surface of the second outermost layer. Thus, the layer-to-layer stop aid adopts a Z-like shape configuration when the structure is in its erected configuration.

Attachment of the layer-to-layer stop aid (180) to the first and second outermost layer (110, 120) in the first and second layer-to-layer stop aid attachment regions (181, 182) can be obtained by any means known in the art, such as adhesive, thermal bonding, mechanical bonding (e.g. pressure bonding), ultrasonic bonding, or combinations thereof.

The layer-to-layer stop aid (180) may be provided in combination with another stop aid, such as with the layer-to-ligament stop aid (190) described below. However the layer-to-layer stop aid (180) alone is normally sufficient to define the maximum shifting of the first layer (110) and the second outermost layer (120) relative to each other in opposite longitudinal directions upon application of a force along the longitudinal dimension.

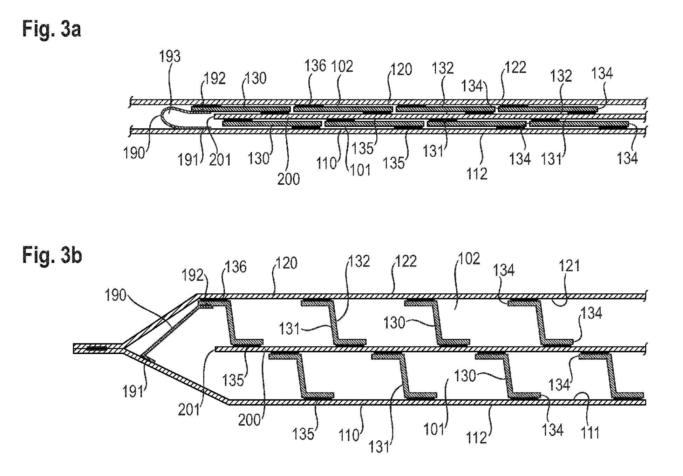

c) A layer-to-ligament stop aid (190) may be provided, which extends from the first or second outermost layer (110, 120) to one of the ligaments (130) comprised by the level which is farthest away from the respective first or second outermost layer in the direction perpendicular to the longitudinal and lateral dimensions (i.e. in caliper direction). This layer-to-ligament stop aid (190) is attached to the inner surface (111) or outer surface (112) of the first or second outermost layer (110, 120) in a first layer-to-ligament stop aid attachment region (191) and is further attached to the first surface (131) or second surface (132) of the ligament (130) in a second layer-to-ligament stop aid attachment region (192). The first layer-to-ligament stop aid attachment region (191) may be longitudinally spaced apart from the second layer-to-ligament stop aid attachment region (192). The layer-to-ligament stop aid (190) is provided with a layer-to-ligament stop aid leeway between the first and second layer-to-ligament stop aid attachment region (191, 192) when the structure (100) is in its initial flat configuration. The leeway may be configured in form of a slack (193). Upon application of a force in the longitudinal dimension the first and second outermost layer (110, 120) shift relative to each other in opposite longitudinal directions, the structure extends and erects, and the slack (193) forming the layer-to-ligament stop aid leeway between the first and second layer-to-ligament stop aid attachment regions (191, 192) straightens out. Once the slack (193) is straightened out when the structure (100) is in its erected position, further longitudinal extension of the structure is inhibited also when the force in the longitudinal dimension is continued to be applied. A layer-to-ligament stop aid (190) is shown in Figs. 3A (initial flat configuration) and 3B (erected configuration).

Alternatively or in addition, the leeway of the layer-to-ligament stop aid (190) can be generated by adapting the material between the first and second layer-to-ligament stop aid attachment regions (191, 192) to create extensibility of the respective area of the layer-to-ligament stop aid (190). Adapting the material can be done by modifying the material, e.g. by selfing (weakening the material of the first or second layer to render it relatively easily extensible), creating holes or using extensible materials to form the leeway.

Alternatively or in addition, the layer-to-ligament stop aid (190) may be made of extensible material. For such layer-to-ligament stop aids (190), the material of the leeway elongates when the first and second outermost layers (110, 120) are shifted against each other until elongation of the layer-to-ligament stop aid leeway is not possible any longer (without applying an excessive amount of force, which may even rupture the structure). Hence, the material in the leeway has reached its maximum elongation i.e. it cannot be elongated further upon application of force without causing damage to the structure that limits or impedes its intended use.

The material of the leeway may also be elastic. For such structures, the layer-to-ligament stop aid (190) can retract when the force is no longer applied onto the structure such that the structure can substantially "snap back" into its initial flat configuration.

For the material properties and appropriate selection of extensible or elastic leeways, the same considerations apply as are set out above for the layer-on-layer stop aid.

It is also possible to provide a leeway that is a combination of a slack and the provision of extensible material in the leeway, such that, upon elongation, initially the slack straightens out and subsequently, the extensible material elongates.

Attachment of the layer-to-ligament stop aid (190) to the first and second outermost layer (110, 120) in the first and second layer-to-layer stop aid attachment regions (181, 182) can be obtained by any means known in the art, such as adhesive, thermal bonding, mechanical bonding (e.g. pressure bonding), ultrasonic bonding, or combinations thereof.

The layer-to-ligament stop aid (190) may be provided in combination with another stop aid, such as with the layer-to-ligament stop aid (190) or with the layer-on-layer stop aid as are described below. However the layer-to-ligament stop aid (190) alone is normally sufficient to define the maximum shifting of the first layer (110) and the second outermost layer (120) relative to each other in opposite longitudinal directions upon application of a force along the longitudinal dimension.

Generally, the layer-to-ligament stop aid (190) may be attached in the first and second layer-to-ligament stop aid attachment regions such that the layer-to-ligament stop aid extends along or adjacent to one of the longitudinal edges of the at least one ligament (130) or, alternatively, such that it extends between the longitudinal edges of the at least one ligament.

d) The structure (100) may comprise an enveloping stop aid (not shown) which encircles at least a portion of the first and second outermost layer (110, 120), the intermediate layer(s) and the ligaments (130) provided in the respective portion. This enveloping stop aid is attached to the first outermost layer (110), the second outermost layer (120), one or more of the intermediate layer(s) and/or at least one of the ligaments (130) in one or more enveloping stop aid attachment region. Attaching the enveloping stop aid to only one of the first outermost layer (110), the second outermost layer (120), one of the intermediate layer(s) or at least one of the ligaments (130) in only one enveloping stop aid attachment region is, however, sufficient.

The enveloping stop aid is attached to itself to form a closed loop with a defined circumference around at least a portion of the first and second outermost layer with the intermediate layer(s) and ligaments in between. The enveloping stop aid may encircle the first and second outermost layer (110, 120) along the longitudinal dimension or along the lateral dimension. Generally, if the enveloping stop aid encircles the first and second outermost layer (110, 120) along the lateral dimension, the risk of the enveloping stop aid sliding off the first and second outermost layer (110, 120) upon elongation and erection of the structure may be lower compared to the enveloping stop aid encircling the first and second outermost layer along the longitudinal dimension, especially for rather long structures. However, by providing further enveloping stop aid attachment regions, such risk can be reduced.

The circumference of the enveloping stop aid defines the maximum shifting of the first outermost layer (110) and the second outermost layer (120) relative to each other in opposite longitudinal directions upon application of a force along the longitudinal dimension.

When the structure (100) is in its initial flat configuration, the enveloping stop aid is loose around the first and second outermost layer (and the respective ligaments between the first and second layer). Upon application of a force along the longitudinal dimension of the structure, the structure erects until the enveloping stop aid fits tightly around the first and second outermost layer (and the respective intermediate layer(s) and ligaments between the first and second outermost layer), which will stop further shifting of the first outermost layer relative to the second outermost layer also if the force along the longitudinal dimension is continued to be applied. To assist in avoiding overexpansion of the structure (100), the circumference of the enveloping stop aid may be such that further shifting of the first outermost layer (110) relative to the second outermost layer (120) along the longitudinal dimensions is inhibited before the ligaments (130) are in their fullest upright position.

*General considerations for the layer-to-layer stop aid, the layer-to-ligament stop aid and, if expressly mentioned, the enveloping stop aid:*

The layer-to-layer stop aid and/or the layer-to-ligament stop aid may be non-elastic or highly non-elastic (apart from the leeway, if the leeway is provided by modifying the material to render it elastically extensible). Also the layer-to-layer stop aid and/or the layer-to-ligament stop aid may be non-extensible or highly non-extensible (apart from the leeway, if the leeway is provided by modifying the material to render it extensible).

The layer-to-layer stop aid and/or the layer-to-ligament stop aid and/or enveloping stop aid can be made of a sheet-like material, such as nonwoven, film, paper, sheet-like foam, woven fabric, knitted fabric, or combinations of these materials. Combinations of these materials may be laminates, e.g. a laminate of a film and a nonwoven. The layer-to-layer stop aid and/or the layer-to-ligament stop aid and/or enveloping stop aid may also be made of a cord- or string-like material.

The layer-to-layer stop aid and/or the layer-to-ligament stop aid and/or enveloping stop aid is not necessarily intended to contribute to the resistance of the structure against a force exerted onto the structure in the thickness-direction. However, the basis weight, tensile strength and bending stiffness of the layer-to-layer stop aid and/or the layer-to-ligament stop aid and/or enveloping stop aid should be sufficiently high to avoid inadvertent tearing of the layer-to-layer stop aid and/or the layer-to-ligament stop aid and/or enveloping stop aid upon expansion of the structure.

If the layer-to-layer stop aid and/or the layer-to-ligament stop aid and/or enveloping stop aid is made of a sheet-like material, the basis weight of the layer-to-layer stop aid and/or the layer-to-ligament stop aid and/or enveloping stop aid may be at least 1 $g/m^2$, or at least 2 $g/m^2$, or at least 3 $g/m^2$, or at least 5 $g/m^2$; and the basis weight may further be not more than 500 $g/m^2$, or not more than 200 $g/m^2$, or not more than 100 $g/m^2$, or not more than 50 $g/m^2$, or not more than 30 $g/m^2$.

If the layer-to-layer stop aid and/or the layer-to-ligament stop aid and/or enveloping stop aid is made of a cord- or string-like material, the basis weight of the layer-to-layer stop aid and/or the layer-to-ligament stop aid and/or enveloping stop aid may be at least 1 gram per meter (g/m), or at least 2 g/m, or at least 3 g/m, or at least 5 g/m; and the basis weight may further be not more than 500 g/m, or not more than 200 g/m, or not more than 100 g/m, or not more than 50 g/m, or not more than 30 g/m.

The basis weight of the layer-to-layer stop aid and/or the layer-to-ligament stop aid may be less than the basis weight of the ligaments, for example the basis weight of the layer-to-layer stop aid and/or the layer-to-ligament stop aid may be less than 80%, or less than 50% of the basis of the ligaments (if the ligaments vary in basis weight, then these values are with respect to the ligament(s) with the lowest basis weight).

The tensile strength of the layer-to-layer stop aid may be at least 2 N/cm, or at least 4 N/cm or at least 5 N/cm.

The tensile strength may be less than 100 N/cm, or less than 80 N/cm, or less than 50 N/cm, or less than 30 N/cm, or less than 20 N/cm.

The bending stiffness of the layer-to-layer stop aid and/or the layer-to-ligament stop aid and/or enveloping stop aid may be at least 0.1 mNm, or at least 0.2 mNm, or at least 0.3 mNm. The bending stiffness may be less than 200 mNm, or less than 150 mNm, or less than 100 mNm, or less than 50 mNm, or less than 10 mNm, or less than 5 mNm. These values apply to sheet-like layer-to-layer stop aids and/or layer-to-ligament stop aids and/or enveloping stop aids, for cord- or string-like layer-to-layer stop aids and/or layer-to-ligament stop aids and/or enveloping stop aids, the bending stiffness is generally not seen as critical.

The tensile strength of the layer-to-layer stop aid and/or the layer-to-ligament stop aid and/or enveloping stop aid may be lower than the tensile strength of the ligaments, for example the tensile strength of the layer-to-layer stop aid may be less than 80%, or less than 50% of the tensile strength of the ligaments (if the ligaments vary in tensile strength, then these values are with respect to the ligament(s) with the lowest tensile strength).

The bending stiffness of the layer-to-layer stop aid and/or the layer-to-ligament stop aid and/or enveloping stop aid (when made of sheet-like material) may be lower than the bending stiffness of the ligaments, for example the bending stiffness of the layer-to-layer stop aid and/or the layer-to-ligament stop aid and/or enveloping stop aid may be less than 80%, or less than 50% of the bending stiffness of the ligaments (if the ligaments vary in bending stiffness, then these values are with respect to the ligament(s) with the lowest bending stiffness).

Alternatively or in addition to the provision of a stop aid comprised by the structure, the maximum possible elongation and erection of the structure can also be determined by a means that is comprised by the disposable consumer product (such as an absorbent article). Such means does not need to be in direct contact with the structure. For example, when the structure is provided by a waistband of an absorbent article, a means acting like a stop aid may be provided in proximity to the structure. Upon application of a force along the transverse direction of the absorbent article, the structure elongates and erects. Simultaneously, a piece of extensible or elastic material provided adjacent to the structure may elongate until it has reached its maximum elongation i.e. it cannot be elongated further upon application of force without causing damage to the structure that limits or impedes its intended use, thus preventing the structure from being elongated futher. Such feature can also be provided in proximity to the structure by a piece of (non-extensible and non-elastic) material facilitated with a slack, which straightens out.

**Partial stop aid**

It is also possible to provide partial stop aids. A partial stop aid can be provided between one of the first and second outermost layer (110, 120) and one of the intermediate layers (a layer-to-layer *partial* stop aid). Alternatively, a partial stop aid can be provide between one of the first and second layer (110, 120) and one of the ligaments which is comprised by a level which is not farthest away from the respective first or second outermost layer in the direction perpendicular to the longitudinal and lateral structure dimension (a layer-to-ligament *partial* stop aid).

A layer-to-layer *partial* stop aid only differs from a layer-to-layer stop aid with respect to the position of the layer to which it is attached in the second attachment region. Hence a layer-to-layer stop aid is attached to the second outermost layer (in addition to being attached to the first outermost layer) while a layer-to-layer partial stop aid is attached to one of the intermediate layer(s) (in addition to being attached to the first outermost layer). Apart from this difference, all other considerations which are set out above for the layer-to-layer stop aid similarly apply to a layer-to-layer partial stop aid, (such as attachment regions, configuration of attachment, provision of leeway, suitable material and material properties).

A layer-to-ligament *partial* stop aid only differs from a layer-to-ligament stop aid with respect to the position of the ligament to which it is attached. Hence a layer-to-ligament stop aid is attached to a ligament comprised by the level farthest away from the respective first or second layer in the direction perpendicular to the longitudinal and lateral structure dimension while a layer-to-ligament partial stop aid is attached to a ligament of another level. Apart from this difference, all other considerations which are set out above for the layer-to-ligament stop aid similarly apply to a layer-to-ligament partial stop aid, (such as attachment regions, configuration of attachment, provision of leeway, suitable material and material properties).

A partial stop aid acts to define a maximum caliper of the erected structure while not defining the maximum possible elongation. When the partial stop aid has straightened and flattened out (when comprising leeways provided as a slack) and/or when the partial stop aid has reached its maximum elongation in the absence of excessive forces applied (when comprising leeways provided by extensible or elastic material), some of the levels will not be able to shift relative to each other in opposite directions any longer. However, one or more other levels can still continue to shift, thus overturning the ligaments comprised by these levels. Thereby, the structure continues to elongate until the ligaments comprised by the respective level(s) are turned over and the respective level(s) are in their final flat position. The caliper of the structure in its maximum elongated state is thus smaller than the caliper of the structure in the stage when the partial stop aid(s) have just reached their maximum extension/flattening out. The partial stop aids, upon further elongation of the structure after the maximum caliper has been obtained, will not change their shape/length any more.

Partial stop aids may be desirable, if a gap formed in use of a disposable consumer product (such as an absorbent article, a bandage or wound dressing) e.g. between the product and the skin of the wearer, shall initially be filled by the erected structure. However, at a certain stage of stretching and extension of the consumer product, it may be desirable to rather reduce the caliper again, e.g. in order not to exert excessive pressure on the body of a wearer.

**Disposable absorbent articles**

The structures of the present invention can find a wide variety of applications in absorbent articles.

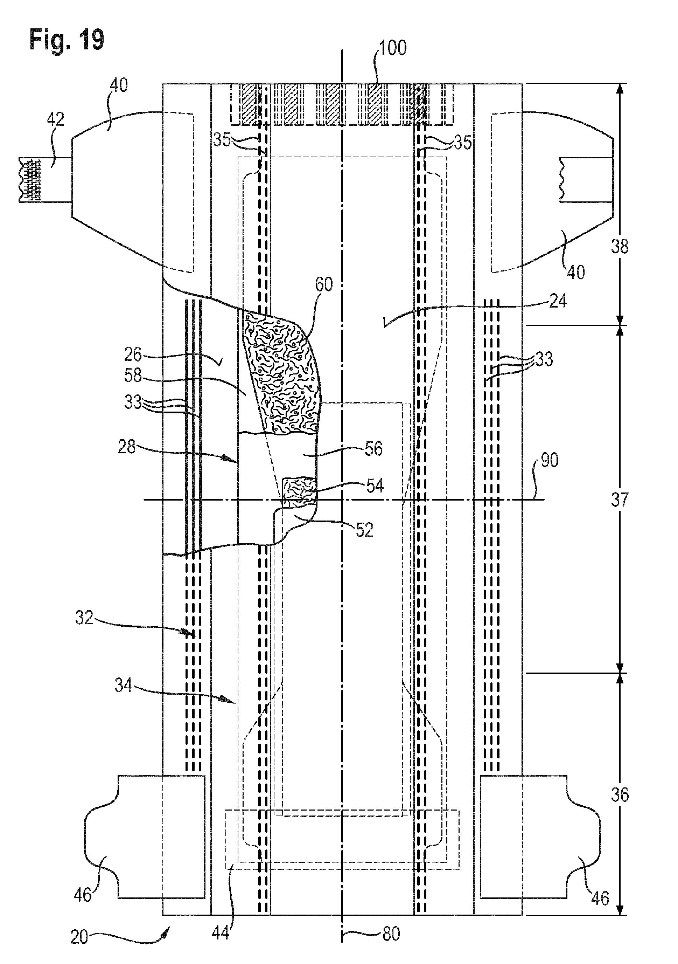

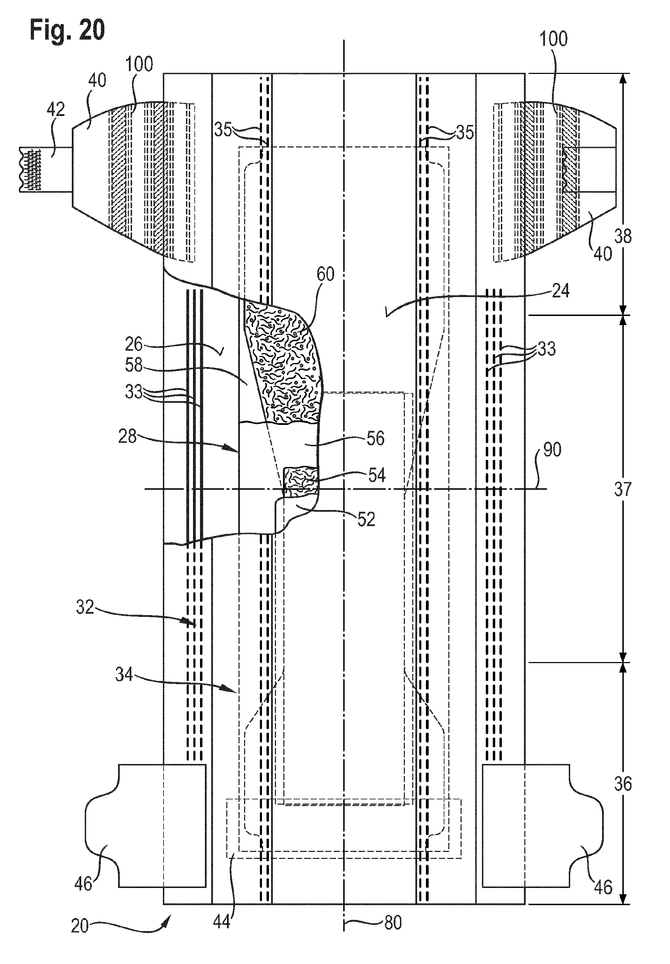

A typical disposable absorbent article of the present invention is represented in Fig. 19 and 20 in the form of a diaper 20.

In more details, Figures 19 and 20 is a plan view of an exemplary diaper 20, in a flat-out state, with portions of the diaper being cut-away to more clearly show the construction of the diaper 20. This diaper 20 is shown for illustration purpose only as the structure of the present invention may be comprised in a wide variety of diapers or other absorbent articles.

As shown in Figures 19 and 20, the absorbent article, here a diaper, can comprise a liquid pervious topsheet 24, a liquid impervious backsheet 26, an absorbent core 28 which is preferably positioned between at least a portion of the topsheet 24 and the backsheet 26. The absorbent core 28 can absorb and contain liquid received by the absorbent article and may comprise absorbent materials 60, such as superabsorbent polymers and/or cellulose fibers, as well as other absorbent and non-absorbent materials commonly used in absorbent articles (e.g. thermoplastic adhesives immobilizing the superabsorbent polymer particles). The diaper 20 may also include optionally an acquisition system with an upper 52 and lower 54 acquisition layer.

The diaper may also comprise elasticized leg cuffs 32 and barrier leg cuffs 34, and a fastening system, such as an adhesive fastening system or a hook and loop fastening member, which can comprise tape tabs 42, such as adhesive tape tabs or tape tabs comprising hook elements, cooperating with a landing zone 44 (e.g. a nonwoven web providing loops in a hook and loop fastening system). Further, the diaper may comprise other elements, such as a back elastic waist feature and a front elastic waist feature, side panels or a lotion application.

The diaper 20 as shown in Figures 19 and 20 can be notionally divided in a first waist region 36, a second waist region 38 opposed to the first waist region 36 and a crotch region 37 located between the first waist region 36 and the second waist region 38. The longitudinal centerline 80 is the imaginary line separating the diaper along its length in two equal halves. The transversal centerline 90 is the imagery line perpendicular to the longitudinal line 80 in the plane of the flattened out diaper and going through the middle of the length of the diaper. The periphery of the diaper 20 is defined by the outer edges of the diaper 20. The longitudinal edges of the diaper may run generally parallel to the longitudinal centerline 80 of the diaper 20 and the end edges run between the longitudinal edges generally parallel to the transversal centerline 90 of the diaper 20.

The majority of diapers are unitary, which means that the diapers are formed of separate parts united together to form a coordinated entity so that they do not require separate manipulative parts like a separate holder and/or liner.

The diaper 20 may comprise other features such as back ears 40, front ears 46 and/or barrier cuffs 34 attached to form the composite diaper structure. Alternatively, the front and/or back ears 40, 46 may not be separate components attached to the diaper but may instead be continuous with the diaper, such that portions of the topsheet and/or backsheet -and even portions of the absorbent core - form all or a part of the front and/or back ears 40, 46. Also combinations of the aforementioned are possible, such that the front and/or back ears 40, 46 are formed by portions of the topsheet and/or backsheet while additional materials are attached to form the overall front and/or back ears 40, 46.

The topsheet 24, the backsheet 26, and the absorbent core 28 may be assembled in a variety of well known configurations, in particular by gluing or heat embossing. Exemplary diaper configurations are described generally in US3,860,003; US5,221,274; US5,554,145; US5,569,234; US5,580,411; and US6,004,306.

The diaper 20 may comprise leg cuffs 32 and/or barrier cuffs 34 which provide improved containment of liquids and other body exudates especially in the area of the leg openings. Usually each leg cuff 32 and barrier cuff 34 will comprise one or more elastic string 33 and 35, represented in exaggerated form on Figs. 19 and 20.

The structure of the present invention may be comprised e.g. by the front and/or back waist feature of an absorbent article, e.g. by the front and/or back waistband.

As the structure has a relatively low caliper when in its initial flat configuration, the volume and bulk of the diaper before use is not significantly increased when using the structure as a component in an absorbent article. Hence, the structures do not add significantly to the overall packaging and storage volume of the absorbent articles. In use, when the caretaker or user handles the absorbent article such that a force is applied to the structure along the longitudinal dimension of the structure, the structure elongates in the longitudinal direction and erects. Upon release of the force, the structure may return essentially to its initial flat configuration, and thus, the structure exhibits an elastic-like behavior.

The structure of the present invention may be comprised e.g. by the back waist feature (such as the back waistband) of an absorbent article such that the longitudinal dimension of the structure is substantially parallel with the transversal centerline of the absorbent article. "Substantially parallel" means that the longitudinal dimension of the structure does not deviate by more than 20°, or by more than 10°, or by more than 5° from the lateral centerline of the absorbent article. The structure may further be applied such that the lateral dimension of the structure is substantially parallel to the longitudinal centerline of the absorbent article. "Substantially parallel" means that the lateral dimension of the structure does not deviate by more than 20°, or by more than 10°, or by more than 5° from the longitudinal centerline of the absorbent article. One of the structures lateral longitudinal edges may coincide with the end edge of the back waist region. Alternatively, the structure may be applied more inboard towards the lateral centerline. In these embodiments, the structure may be positioned to form a distance between the absorbent articles end edge of the back waist region and the longitudinal edge of the structure being closest to the respective end edge of from 0.5 cm to 20 cm, or from 0.5 cm to 15 cm, or from 0.5 cm to 10 cm, or from 1 cm to 5 cm. Larger distances, such as 20 cm, may be especially applicable for diapers or pants to be worn by adults (which generally have considerably larger size and dimensions than diapers and pants for baby and toddlers).An embodiment wherein the structure is used as a waistband positioned at the end edge of the absorbent article's back waist region is shown in Fig. 19.

When the absorbent article is in an untensioned state, e.g. when the absorbent article is in a package, the structure is in its initial flat configuration. When the caretaker or wearer applies a force along the longitudinal direction of the structure (e.g. by pulling the article in the waist region parallel to the lateral centerline of the absorbent article to apply the absorbent article around the waist of the wearer), the structure is extended along its longitudinal dimensions and is converted into its erected configuration. This provides a snug fit of the article around the waist of the wearer and ensures that gaps which may potentially be formed between the skin of the wearer and the article is kept to a minimum. Especially, if the structure has not been erected to its maximum caliper upon application of the absorbent article onto a wearer, any subsequent further expansion of the absorbent article around the waist area, e. g. due to movement of the wearer, such as bending or leaning forward, can lead to a further expansion of the structure along the longitudinal dimension and at the same time can also lead to a further increase in caliper of the structure. Hence, e.g. a gap, which typically forms in the back waist area between the absorbent article and the skin of the wearer, upon leaning forward, is closed (at least to some extent) by the increase in caliper of the structure.

When the structure is comprised by any of the front waist feature (e.g. as a front waistband), the front ears, the back ears, the tape tabs, the landing zone of an absorbent article or combinations thereof, the risk of folding over outwardly (i.e. away from the wearer's skin) of the article during use can be reduced. The structure can be applied such that the longitudinal dimension of the structure is substantially parallel to the lateral centerline of the absorbent article. "Substantially parallel" means that the longitudinal dimension of the structure does not deviate by more than 20°, or by more than 10°, or by more than 5° from the lateral centerline of the absorbent article. The structure may further be applied such that the lateral dimension of the structure is substantially parallel to the longitudinal centerline of the absorbent article. "Substantially parallel" means that the lateral dimension of the structure does not deviate by more than 20°, or by more than 10°, or by more than 5° from the longitudinal centerline of the absorbent article.

When comprised by the tape tabs, the tape tabs can be rendered softer compared to the relatively stiff film materials which are often used for making tape tabs. At the same time, sufficient stability of the tape tab is provided, as the tape tab has low tendency to fold over.

When used as a front waistband, one of the structures lateral longitudinal edges may coincide with the end edge of the front waist region. Alternatively, the structure may be applied more inboard towards the lateral centerline. In these embodiments, the structure may be positioned to form a distance between the absorbent articles end edge of the front waist region and the longitudinal edge of the structure being closest to the respective end edge of from 0.5 cm to 30 cm, or from 0.5 cm to 25 cm, or from 0.5 cm to 15 cm, or from 1 cm to 10 cm. Larger distances, such as 20 cm or larger, may be especially applicable for diapers or pants to be worn by adults (which generally have considerably larger size and dimensions than diapers and pants for baby and toddlers).

The positioning and dimensions given in the previous paragraph likewise apply when the structure is comprised by the front and/or back ears. If such structure is in an erected configuration, the upper edges of the front waist region and/or the sides of the absorbent article in the area of the front and/or back ears, have a reduced tendency to fold over outwardly (e.g. when the wearer leans forward), because the erected structure provides increased stiffness along the longitudinal direction of the absorbent article (and hence, in the lateral dimension of the structure). At the same time, the elastic-like behavior of the structure enables proper fit around the waist area of the wearer (hence, along the longitudinal dimension of the structure). Also, as the structure erects upon elongation in the longitudinal dimension, a snug contact between the absorbent article and the skin of the wearer can be provided. It may also serve as a feedback mechanism that the maximum extension of the flexible ear and/or waist feature is reached upon application of a force by the care taker as it provides a tactile signal that the maximum elongation of the feature is reached. This may not only provide better control but also helps to avoid damaging of weaker materials that are in the same or similar line of tensioning as the cell forming structure. An example of an absorbent article, wherein the structure is comprised by the back ears, is shown in Fig. 20.

The front and/or back waist feature may be provided between the topsheet and the backsheet of the absorbent article, respectively. Alternatively, the front and/or back waist feature may be provided on the topsheet towards the skin of the wearer, when the article is in use. In another alternative, the front and/or back waist feature may be provided on the backsheet towards the garments of the wearer, when the article is in use.

When the structure is comprised by the front and/ or back waist feature, the respective portions of the topsheet may form the first outermost layer of the structure. In addition, or alternatively, the respective portions of the backsheet may form the second outermost layer of the structure.

Similar, when the structure is comprised by the front and/ or back ears, one or more layers of the respective portions of the front and/or back ear may form the first outermost layer of the structure. In addition, or alternatively, one or more other layers of the respective portions of the front and/or back ear may form the second outermost layer of the structure.

When the structure is comprised by the front and/or back waistband, the structure may extend across the complete lateral dimension of the absorbent article - including the front and/or back ears. Alternatively, the structure may extend only across a part of the lateral dimension of the absorbent article (either extending onto the front and/or back ears or not). Also, more than one structure may be comprised by each of the front and/or back waistband. These structures may be provided adjacent to each other across the lateral dimension of the absorbent article, and these structures may or may not be provided with a gap between them.

When the structure is comprised by the front and/or back waist feature, the structure may extend across the complete lateral dimension of the backsheet at or adjacent to the front waist edge of the absorbent article and/or the back waist edge of the absorbent article. Alternatively, the structure may extend only across a part of the lateral dimension of the backsheet at or adjacent to the front waist edge of the absorbent article and/or the back waist edge of the absorbent article.

Also, when comprised by a waist feature the structure may extend fully or partly into the front and/or back ears. A continuous structure may be applied across the lateral dimension of the backsheet extending fully or partly into the front and/or back ears. Alternatively, one structure may extend partly or fully across the lateral dimension of the backsheet and a separate structure may extend partly or fully across each of the front and/or back ears.

The structure may be comprised by a front and/or back waist feature in combination with a elastic waistband, such as those well known in the art. That way, the elastic waistband can gather the front and/or back waist area. In use, the elastic waistband extends, the gathers in the front and/or back waist area straighten out and thus, the structure, which is likewise attached to the respective front and/or back waist area, elongates and erects. The erected structure can then help to fill possible gaps otherwise formed between the absorbent article and the skin of the wearer.

It may also be desirable to facilitate the structure with an elastic stop aid, such as with an elastic leeway of a layer-on-layer stop aid, as is describe above. It may be especially desirable to provide such elastic leeway of a layer-on-layer stop aid towards at least one of the lateral edges of the structure. If the absorbent article, such as a diaper, is applied onto the wearer while the wearer is lying on his or her back, at least a portion of the back waist area may be obstructed from extending laterally outward due to the weight of the wearer. By tensioning the diaper along the lateral dimension when applying and fastening the absorbent article around the waist of the wearer, the elastic leeway of the layer-on-layer stop aid is stretched out and extended. When the wearer lifts up his or her back after the absorbent article has been applied, a part of the tension in the elastic leeway is distributed more evenly over the lateral dimension of the absorbent article, thereby causing the structure to elongate and erect.

In a pant, wherein the front and back waist regions are attached to each other to form leg openings, the structure may encircle the complete waist opening or may, alternatively, span only a portion of the waist opening, such as the waist opening formed by the back waist region or by the front waist region. The structure is attached to the absorbent article such that extension of the structure along its longitudinal dimension and simultaneous conversion from its initial flat configuration into its erected configuration is not hindered due to inappropriate attachment of the structure, or parts thereof, to other components of the absorbent article.

To appropriately incorporate the structure into or onto an absorbent article, it may be sufficient to attach the first and second outermost layer of the structure at or adjacent their lateral edges to other components of the absorbent article while leaving the remaining parts of the structure unattached to any other components of the absorbent article. For example, when the topsheet and backsheet of an absorbent article are attached to each other along their longitudinal edges in the front and back waist region, the areas at or adjacent the lateral edges of the first and second outermost layer of the structure may be attached between the backsheet and the topsheet in these topsheet to backsheet attachment regions. If the structure is attached towards the garment-facing surface of the backsheet, the areas at or adjacent the lateral edges of the first and second outermost layer of the structure may be attached at or adjacent to the longitudinal edges of the backsheet in the front and/or back waist region. If the structure is attached towards the wearer-facing surface of the topsheet, the areas at or adjacent the lateral edges of the first and second outermost layer of the structure may be attached at or adjacent to the longitudinal edges of the topsheet in the front and/or back waist region.

Also, when the structure extends into the front and/or back ears the areas at or adjacent the lateral edges of the first and second outermost layer of the structure may be attached to the front/and or back ears.

The structure may also be comprised by handles, which are provided in the waist areas of a pant, such as in the areas at or adjacent to the side seams, where the front and back waist regions are attached to each other to form leg openings. The handles help users and caregivers to lift the pants upwardly over the hips of the wearer. By using the structures of the present invention, the handles are flat and hence, less volume-consuming when comprised by a package but are soft while still robust in use.

**Other uses of the structures**

The structures of the present invention can be used in a large variety of consumer products. Examples are wound dressings or bandages. Wound dressings and bandages comprising one or more structures of the present invention, can be held in intimate contact with parts of a human or animal body are with a wound. In addition, the structures of the present invention can provide a buffering effect, acting as antishocks in case the part of a body or wound which are covered by the bandage or wound dressing is unintentionally bounced against a hard surface, due to the ability of the structure to increase in caliper when being elongated.

If one or more structures of the present invention are comprised by a flexible packaging (wherein the flexible packaging may be made of film), the structures can provide a cushioning effect, thus assisting in protecting the contents of the flexible package. Furthermore, a flexible packaging comprising one or more structures of the present invention can fill areas within the packaging which would otherwise be empty due to the shape of the products contained in the packaging. Thereby, the structures can help to balance or avoid packaging deformations. This can allow for e.g. more rectangular packaging shape, which enables easier handling and storage (especially when several packages are stacked upon each other).

**Test methods:**

**Tensile Strength**

Tensile Strength is measured on a constant rate with extension tensile tester Zwick Roell Z2.5 with computer interface, using TestExpert 11.0 Software, as available from Zwick Roell GmbH &Co. KG, Ulm, Germany. A load cell is used for which the forces measured are within 10% to 90% of the limit of the cell. Both the movable (upper) and stationary (lower) pneumatic jaws are fitted with rubber faced grips, wider than the width of the test specimen. All testing is performed in a conditioned room maintained at about 23°C + 2°C and about 45 % $\pm$ 5% relative humidity.

With a die or razor knife, cut a material specimen which is 25.4 mm wide and 100 mm long. For the present invention, the length of the specimen correlates to the longitudinal dimension of the material within the structure.

If the ligament is smaller than the size of the material specimen specified in the previous paragraph, the material specimen may be cut from a larger piece such as the raw material used for making the ligaments. Care should be taken to correlate the orientation of such specimen accordingly, i.e. with the length o the specimen correlating to the longitudinal dimension of the material within the structure. However, if the ligament has a width somewhat smaller than 25.4 mm (e.g. 20 mm, or 15 mm) the width of the specimen can be accordingly smaller without significantly impacting the measured tensile strength.

If the ligament comprises different materials in different regions, the tensile strength of each material can be determined separately by taking the respective raw materials. It is also possible to measure the tensile strength of the overall ligament. However, in this case, the measured tensile test will be determined by the material within the ligament which has the lowest tensile strength.

Precondition the specimens at about 23 °C $\pm$ 2°C and about 45 % $\pm$ 5% relative humidity for 2 hours prior to testing.

For analyses, set the gauge length to 50 mm. Zero the crosshead and load cell. Insert the specimen into the upper grips, aligning it vertically within the upper and lower jaws and close the upper grips. Insert the specimen into the lower grips and close. The specimen should be under enough tension to eliminate any slack, but less than 0.025 N of force on the load cell.

Program the tensile tester to perform an extension test, collecting force and extension data at an acquisition rate of 50 Hz as the crosshead raises at a rate of 100 mm/min until the specimen breaks. Start the tensile tester and data collection. Program the software to record Peak Force (N) from the constructed force (N) verses extension (mm) curve. Calculate tensile strength as:

$$\text{Tensile Strength} = \text{Peak Force (N)} / \text{width of specimen (cm)}$$

For rope/string like materials: tensile strength = peak force (N)

Analyze all tensile Specimens. Record Tensile Strength to the nearest 1 N/cm. A total of five test samples are analyzed in like fashion. Calculate and report the average and standard deviation of Tensile Strength to the nearest 1 N/cm for all 5 measured specimens.

**Bending Stiffness**

Bending stiffness is measured using a Lorentzen & Wettre Bending Resistance Tester (BRT) Model SE016 instrument commercially available from Lorentzen & Wettre GmbH, Darmstadt, Germany. Stiffness off the materials (e.g. ligaments and first and second layer) is measured in accordance with SCAN-P 29:69 and corresponding to the requirements according to DIN 53121 (3.1 "Two-point Method"). For analysis a 25.4 mm by 50 mm rectangular specimen was used instead of the 38.1 mm by 50 mm specimen recited in the standard. Therefore, the bending force was specified in mN and the bending resistance was measured according to the formula present below.

The bending stiffness is calculated as follows:

$$S_b = \frac{60 \times F \times l^2}{\pi \times \alpha \times b}$$

with:

$S_b$ = bending stiffness in mNm
F = bending force in N
1 = bending length in mm
$\alpha$ = bending angle in degrees
b = sample width in mm

With a die or razor knife, cut a specimen of 25.4 mm by 50 mm whereby the longer portion of the specimen corresponds to the lateral dimension of the material when incorporated into a structure. If the materials are relatively soft, the bending length "1" should be 1 mm. However, if the materials are stiffer such that the load cell capacity is not sufficient any longer for the measurement and indicates "Error", the bending length "1" has to be set at 10 mm. If with a bending length "1" of 10 mm, the load cell again indicates "Error", the bending length "1" may be chosen to be more than 10 mm, such as 20 mm or 30 mm. Alternatively (or in addition, if needed), the bending angle may be reduced from 30° to 10°.

For the ligament data given in Table 1, the bending length "1" was 10 mm. For the 1st and 2nd layer materials the bending length "1" was 1 mm. The bending angel has been 30° for the ligaments as well as for the 1st and 2nd outermost layers and intermediate layers.

Precondition the specimen at about 23 °C $\pm$ 2°C and about 45 % $\pm$ 5% relative humidity for two hours prior to testing.

Regarding the size of the ligament and the procedure in case the size of the ligament is smaller than the size of the specimen, the same applies as set out above for the tensile test.

**Table 1:** Basis weight, tensile strength and bending stiffness of materials suitable as first and second outermost layers and intermediate layers:

| Example No. | 1 | 2 | 3 | 4 |
|---|---|---|---|---|
| Basis weight | 13 g/m$^2$ | 15 g/m$^2$ | 17 g/m$^2$ | 25 g/m$^2$ |
| Bending stiffness | 0.3 mNm | 0.4 mNm | 0.5 mNm | 0.9 mNm |
| Tensile strength | 6.9 N/cm | 7.9 N/cm | 7.8 N/cm | 8.1 N/cm |

Example materials 1 to 4 are all spunbond polypropylene nonwovens.

**Table 2:** Basis weight, tensile strength and bending stiffness of suitable ligament materials:

| Example No. | 1 | 2 | 3 | 4 |
|---|---|---|---|---|
| Basis weight of ligaments | 40 g/m$^2$ | 43 g/m$^2$ | 51 g/m$^2$ | 60 g/m$^2$ |
| Bending stiffness of ligaments | 10.8 mNm | 36.3 mNm | 52.6 mNm | 105.3 mNm |
| Tensile strength of ligaments | 16.2 N/cm | 16.1 N/cm | 18.8 N/cm | 26.1 N/cm |

Except for Example 1 (40 g/m$^2$ material), all ligament materials were spunbond PET nonwovens. Example 1 was a spunbond polypropylene material.

**Method to measure ligament caliper**

Average Measured caliper is measured using a Mitutoyo Absolute caliper device model ID-C1506, Mitutoyo Corp., Japan. The caliper of the cell forming structure was measured in accordance with EDANA 30.5-99 test method.
A sample of the material used for the ligaments with a sample size of 40 mm x 40 mm is cut. If the samples are taken from a ready-made structure and the size of the ligaments is smaller than 40 mm x 40 mm, the sample may be assembled by placing two or more ligaments next to each other with no gap and no overlap between them. Precondition the specimens at about 23 °C $\pm$ 2°C and about 45 % $\pm$ 5% relative humidity for 2 hours prior to testing.
Place the measuring plate on the base blade of the apparatus. Zero the scale when the probe touches the measuring plate (Measuring plate 40 mm diameters, 1.5 mm height and weight of 2.149 g). Place the test piece on the base plate. Place the measurement plate centrally on top of the sample without applying pressure. After 10 sec. move the measuring bar downwards until the probe touches the surface of the measuring plate and read the caliper from the scale to the nearest 0.01 mm.

**Method to measure caliper of the multilevel structure**

Average Measured caliper is measured using a Mitutoyo Absolute caliper device model ID-C1506, Mitutoyo Corp., Japan.
Precondition the sample multilevel structure at about 23 °C $\pm$ 2°C and about 55 % $\pm$ 5% relative humidity for 2 hours prior to testing.
Place the measuring plate on the base blade of the apparatus. Zero the scale when the probe touches the measuring plate (Measuring plate 40 mm diameters, 1.5 mm height and weight of 2.149 g). Place the multilevel structure (in its flat configuration) on the base plate centrally under the probe position. Place the measurement plate centrally on top of the sample without applying pressure. After 10 sec. move the measuring bar downwards until the probe touches the surface of the measuring plate and read the caliper of the flat structure from the scale to the nearest 0.01 mm.
Transform the multilevel structure into its erected configuration and fix it in its erected configuration with substantially maximum possible structure caliper to the base plate at both lateral edges using tape. Place the measurement plate centrally on top of the piece without applying pressure. After 10 sec. move the measuring bar downwards until the probe touches the surface of the measuring plate and read the caliper of the erected structure from the scale to the nearest 0.01 mm. A total of three test samples are analyzed in like fashion

**Method of measuring modulus of the structure**

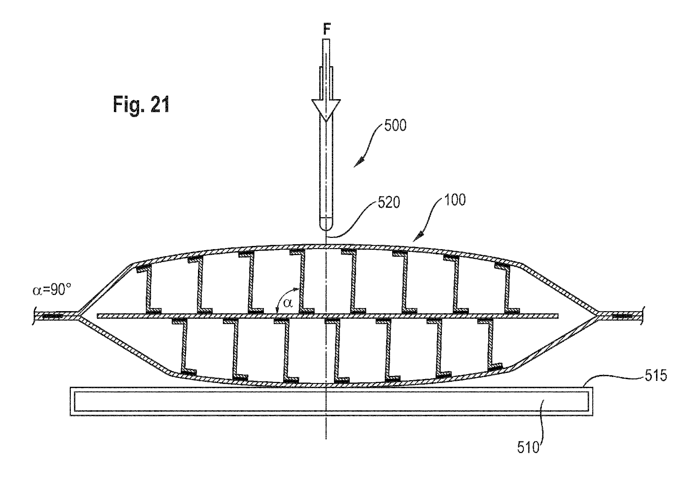

The modulus of the structure is measured on a constant rate of structure compression using a tensile tester with computer interface (a suitable instrument is the Zwick Roell Z2.5 using TestExpert 11.0 Software, as available from Zwick Roell GmbH &Co. KG, Ulm, Germany) using a load cell for which the forces measured are within 10% to 90% of the limit of the cell. The movable upper stationary pneumatic jaw is fitted with rubber faced grip to securely clamp the plunger plate (500). The stationary lower jaw is a base plate (510) with dimensions of 100mm x 100mm. The surface of the base plate (510) is perpendicular to the plunger plate (500). To fix the plunger plate (500) to the upper jaw, lower the upper jaw down to 20mm above the upper surface (515) of the base plate (510). Close the upper jaw and make sure the plunger plate (500) is securely tightened. Plunger plate (500) has a width of 3.2mm and a length of 100mm. The edge (520) of the plunger plate (500) which will contact the structure has a curved surface with an impacting edge radius of r = 1.6mm. For analysis, set the gauge length to at least 10% higher than the caliper of the structure in its erected configuration (see Fig 21). Zero the crosshead and load cell. The width of the plunger plate (500) should be parallel with the transverse direction of the structure.
Precondition samples at about 23 °C $\pm$ 2°C and about 45% RH $\pm$ 5% RH relative humidity for 2 hours prior to testing. The structure is placed on the base plate, is transformed into its erected configuration and fixed in its erected configuration with substantially maximum possible structure caliper to the base plate with the outer surface of its first (lower) outermost layer facing towards the upper surface (515) of the base plate (510). The structure can be fixed to the upper surface of the base plate, e.g. by placing adhesive tapes on the lateral edges of the structure's first (lower) outermost layer and fix the tapes to the upper surface of the base plate.

Program the tensile tester to perform a compression test, collecting force and travel distance data at an acquisition rate of 50 Hz as the crosshead descends at a rate of 50 mm/min from starting position to 2mm above base plate (safety margin to avoid destruction of load cell).

If the modulus of the structure is measured directly in an area where a ligament is placed, the force P [N] is the force when the indentation depth h [mm] of the plunger plate into the structure is equal to 50% of the longitudinal dimension of the free intermediate portion of the ligament below the plunger plate.

If the modulus of the structure is measured between two neighboring ligaments of the uppermost level nearest to the plunger plate, the force P [N] is the force when the indentation depth h [mm] of the plunger plate into the structure is equal to 50% of the longitudinal dimension of the free intermediate portion of these two ligaments (i.e. the ligaments on each side of the plunger plate as seen along the longitudinal structure dimension). If the free intermediate portion of the two neighboring ligaments of the uppermost level nearest to the plunger plate, between which the modulus is measured, differ from each other with respect to the longitudinal dimension of their free intermediate portions, the average value over these two free intermediate portions is calculated and the indentation depth h [mm] of the plunger plate into the structure is equal to 50% of this average free longitudinal dimension.

A total of three test specimens are analyzed in like fashion.

The modulus E [N/mm$^2$] is calculated as follows:

$$E = \frac{3P}{8rh}.$$

With r being the impacting edge radius of the plunger plate, i.e. r = 1.6mm

Calculate and report the average of modulus E for all 3 measured specimens.

All testing is performed in a conditioned room maintained at about 23°C + 2°C and about 45%RH $\pm$ 5% relative humidity.

**Example structures**

Making of Example Structure 1:

Cut two pieces of nonwovens of Example 1 in Table 1, each having a longitudinal dimension of 200 mm and a lateral dimension of 25 mm, with a die or razor knife. These nowovens will become the first and second outermost layers of the Example Structure.

Moreover, cut two pieces of nonwoven of Example 1 given in Table **1** above, each having a longitudinal dimension of 40 mm and a lateral dimension of 25 mm. These nonwovens will become the intermediate layers of Example Structure 1 (i.e. Example Structure 1 has two intermediate layers).

Cut 12 ligaments (for Example Structure 1) of Example 4 nonwoven material given in Table **2** above, each having a longitudinal dimension of 10 mm (which will result in a longitudinal dimension of the free intermediate portion of 4 mm in the final structures, while 3 mm on each lateral edge are attached to the respective first or second outermost layer and intermediate layer) and a lateral dimension of 25 mm, with a die or razor knife. Apply a double sided tape (e.g. 3M Double sided medical tape 1524-3M (44g/m$^2$) available from 3M) with length of 3 mm and width of 25 mm on the first surface of the each ligament adjacent the side edge, which will become the first lateral edge of the ligament in the final structure and a second tape on the second surface of each ligament adjacent the side edge, which will become the second lateral edge of the ligament in the final structure. The width of the tape is aligned with the lateral dimension of the ligaments.

Remove one release tape from four of the ligaments (for Example Structure 1)and attach the tape to the first outermost layer such that the lateral dimension of the ligament is aligned with the lateral dimension of the first layer.

Place the first, second, third and fourth ligaments such that the spacing between neighboring ligaments is 5 mm when the ligaments are lying flat on the first outermost layer.

The ligaments should be positioned accordingly, to leave sufficient space at the lateral edges of the first and second outermost layer to allow attaching the first outermost layer to the second outermost layer in the manner described below.

Remove the remaining release layers from the remaining tape pieces on the ligaments attached to the first outermost layer and place the first intermediate on top of the first outermost layer and the ligaments such that the lateral dimension of the first intermediate layer and of the ligaments is congruent with the lateral dimension of the first outermost layer and such that the first intermediate layer is attached to the remaining tape pieces of the ligaments previously attached to the first outermost layer.

Now attach four ligaments (ligaments of second level) in like fashion to the first intermediate layer on the opposite surface compared to the surface where the first four ligaments have previously been attached. Subsequently, place the second intermediate layer on top of the first intermediate layer and the four ligaments of the second level such that the lateral dimension of the second intermediate layer and of the ligaments is congruent with the lateral dimension of the first outermost layer and such that the second intermediate layer is attached to the remaining tape pieces of the ligaments of the second level which were previously attached to the first intermediate layer.

Attach the remaining four ligaments (ligaments of third level) in like fashion to the second intermediate layer on the opposite surface compared to the surface where the four ligaments of the second level have previously been attached. Subsequently, place the second outermost layer on top of the second intermediate layer and the four ligaments of the third level such that the lateral dimension of the first and second outermost layer, of the first and second intermediate layer and of the ligaments are congruent and such that the second outermost layer is attached to the remaining tape pieces of the ligaments of the third level which were previously attached to the second intermediate layer.

To bond the first outermost layer to the second outermost layer in the areas longitudinally outwardly from the area where the ligaments are placed (stop aid), two double-sided tapes (e.g. 3M Double sided medical tape 1524-3M ($44g/m^2$) available from 3M) having a length of 3 mm and a width of 25 mm are provided. A first tape is attached to the first outermost layer towards one of the first outermost layer's lateral edges such that the distance between this first tape and the lateral edge of the adjacent ligament is 40 mm. The second tape is attached to the first outermost layer towards the respective other lateral edge of the first outermost layer such that the distance between this second tape and the lateral edge of the respective adjacent ligament is 40 mm. The width of the first and second tape is aligned with the lateral dimension of the first outermost layer. Pay attention that the first and second tapes are not attached to the second outermost layer before the structure has been transformed into its erected configuration (see next step).

Extend the resulting multilevel cell forming structure along the longitudinal dimension into the erected configuration such that the first and second outermost layer shift in opposite directions and the ligaments move in upright position of 90° relative to the first and second outermost layer. Notably, the 90° does not apply to the area longitudinally outwardly from the outermost ligaments (viewed along the longitudinal dimension) because the first and second outermost layers follow a tapered path in this area until the point where they coincide with each other (see e.g. Fig. 1B). Maintain the structure in its erected configuration and attach the first outermost layer to the second outermost layer via the first and second tape to fix the structure in its erected configuration. Release the force and allow the structure to relax.

Example Structure 1

## Making of Example Structure 2:

Cut two pieces of nonwovens of Example 1 in Table 1, each having a longitudinal dimension of 200 mm and a lateral dimension of 25 mm, with a die or razor knife. These nowovens will become the first and second outermost layers of the Example Structure.

Moreover, cut one pieces of nonwoven of Example 1 in Table 1, having a longitudinal dimension of 40 mm and a lateral dimension of 25 mm. This nonwoven will become the intermediate layer of Example Structure 2 (i.e. Example Structure 2 has one intermediate layer).

Cut 6 ligaments of Example 4 nonwoven material (see Table 2 above) each having a longitudinal dimension of 10 mm (which will result in a longitudinal dimension of the free intermediate portion of 4 mm in the final structures, while 3 mm on each lateral edge are attached to the respective first or second outermost layer and intermediate layer) and a lateral dimension of 25 mm, with a die or razor knife. Apply a double sided tape (e.g. 3M Double sided medical tape 1524-3M (44g/m$^2$) available from 3M) with length of 3 mm and width of 25 mm on the first surface of the each ligament adjacent the side edge, which will become the first lateral edge of the ligament in the final structure and a second tape on the second surface of each ligament adjacent the side edge, which will become the second lateral edge of the ligament in the final structure. The width of the tape is aligned with the lateral dimension of the ligaments.

Remove one release tape from three of the ligaments and attach the tape to the first outermost layer such that the lateral dimension of the ligament is aligned with the lateral dimension of the first layer.

Place the three ligaments such that the spacing between neighboring ligaments is 5mm when the ligaments are lying flat on the first outermost layer.

The ligaments should be positioned accordingly, to leave sufficient space at the lateral edges of the first and second outermost layer to allow attaching the first outermost layer to the second outermost layer in the manner described below.

Cut 2 pieces of elastic film material with length of 60 mm x 25 mm. The elastic film material is a translucent, colorless latex film available from Modulor GmbH, Berlin, Germany, having a thickness of 0.18 to 0.25 mm and a width of 920 mm (Article Number 813963). The elastic film material has a basis weight of 252 g/m$^2$, a tensile strength of 23.3 N/cm and an Elongation at Break of 859% (tensile strength and Elongation at Break were both determined following the Tensile Strength Test Method set out above).

Place a piece of double side tape with length of 5 mm x 25 mm at each lateral edge of the nonwoven web which will become the first intermediate layer and join one piece of elastic film material on each of the lateral edges of the nonwoven web via the double sided tape, such that one of the shorter edges (25 mm wide) of the elastic film material is attached to the lateral edge of the nonwoven web to form a continuous layer made of elastic film material at its lateral edges and nonwoven web in the center.

Remove the remaining release layers from the tape pieces on the ligaments attached to the first outermost layer and place the first intermediate layer on top of the first outermost layer and the ligaments such that the lateral dimension of the first intermediate layer and of the ligaments is congruent with the lateral dimension of the first outermost layer and such that the central nonwoven web of the first intermediate layer is attached to the remaining tape pieces of the ligaments previously attached to the first outermost layer.

Now attach the remaining three ligaments (ligaments of second level) in like fashion to the first intermediate layer on the opposite surface compared to the surface where the first four ligaments have previously been attached. The ligaments are placed such that they can be attached to the central nonwoven web of the intermediate layer. Subsequently, place the second outermost layer on top of the first intermediate layer and the three ligaments of the second level such that the lateral dimensions of the first and second outermost layer, the intermediate layer and the ligaments are congruent and such that the second outermost layer is attached to the remaining tape pieces of the ligaments of the second level which were previously attached to the first intermediate layer.

To bond the first outermost layer to the second outermost layer in the areas longitudinally outwardly from the area where the ligaments are placed (stop aid), four double-sided tapes (e.g. 3M Double sided medical tape 1524-3M (44g/m$^2$) available from 3M) having a length of 3 mm and a width of 25 mm are provided. A first tape is attached to the first outermost layer towards one of the first outermost layer's lateral edges such that the distance between this first tape and the lateral edge of the adjacent ligament is 40 mm. The second tape is attached to the first outermost layer towards the respective other lateral edge of the first outermost layer such that the distance between this second tape and the lateral edge of the respective adjacent ligament is 40 mm Likewise, the third tape is attached to the second outermost layer towards one of the second outermost layer's lateral edges such that the distance between this first tape and the lateral edge of the adjacent ligament is 40 mm. The fourth tape is attached to the second outermost layer towards the respective other lateral edge of the second outermost layer such that the distance between this second tape and the lateral edge of the respective adjacent ligament is 40 mm. The width of the first, second, third and fourth tape is aligned with the lateral dimension of the first and second outermost layer. Pay attention that the first, second, third and fourth tapes are not attached to the intermediate layer before the structure has been transformed into its erected configuration (see next step).

Extend the resulting multilevel cell forming structure along the longitudinal dimension into the erected configuration such that the first and second outermost layer shift in opposite directions and the ligaments move in upright position of 90° relative to the first and second outermost layer. Notably, the 90° does not apply to the area longitudinally outwardly from the outermost ligaments (viewed along the longitudinal dimension) because the first and second outermost layers follow a tapered path in this area until the point where they coincide with each other (see e.g. Fig. 1B). Maintain the structure in its erected configuration and attach the first outermost layer to one surface of the intermediate layer via the first and second tape and attach the second outermost layer to the respective other surface of the intermediate layer via the third and fourth tape to fix the structure in its erected configuration. Release the force and allow the structure to relax.

Example Structure 2

**Table 3:** Caliper of Example Structures in flat and erected configuration

|  | Example Structure 1 | Example Structure 2 |
|---|---|---|
| Caliper of flat structure | 1.8 mm | 1.2 mm |
| Caliper of erected structure | 13.7 mm | 10.7 mm |

All patents and patent applications (including any patents which issue thereon) assigned to the Procter & Gamble Company referred to herein are hereby incorporated by reference to the extent that it is consistent herewith.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

All documents cited in the Detailed Description of the Invention are, in relevant part, incorporated herein by reference; the citation of any document is not to be construed as an admission that it is prior art with respect to the present invention. To the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1.   A multi-level structure having a longitudinal dimension and a lateral dimension perpendicular to the longitudinal dimension, and comprising at least a first and a second level,
   wherein the multi-level structure comprises a first outermost layer, a second outermost layer, and at least a first intermediate layer,
   all layers having a longitudinal dimension parallel to the longitudinal dimension of the multi-level structure and being confined by two spaced apart lateral edges, and a lateral dimension parallel to the lateral dimension of the multi-level structure and being confined by two spaced apart longitudinal edges, wherein the layers at least partly overlap each other; with the at least first intermediate layer being positioned subjacent the first outermost layer and superjacent the second outermost layer, with each level of the multi-level structure being confined in a direction perpendicular to the longitudinal and lateral dimension by one of the layers forming the superjacent layer of the level and further confined by another of the layers forming the subjacent layer of the level;
   the first and second outermost layer being able to shift relative to each other in opposite directions along the longitudinal multi-level structure dimension upon application of a force along the longitudinal dimension, whereby the multi-level structure is elongated along the longitudinal dimension and simultaneously converted from an initial flat configuration into an erected configuration in a direction perpendicular to the longitudinal and the lateral dimension, each level in the multi-level structure comprising one or more ligament, each ligament having a first surface and a second surface, a longitudinal dimension confined by two spaced apart lateral ligament edges, and a lateral dimension confined by two spaced apart longitudinal ligament edges, each ligament being provided between the superjacent layer and the subjacent layer of the respective level and being attached with a portion at or adjacent to one of the ligament's lateral edges in a first ligament attachment region to the surface of the superjacent layer which faces towards the subjacent layer of the respective level, and being further attached with a portion at or adjacent to the ligament's other lateral edge in a second ligament attachment region to the surface of the subjacent layer which faces towards the superjacent layer of the respective level, the region of each ligament between the first and second ligament attachment region forming a free intermediate portion,

the ligaments in each level being spaced apart from one another along the longitudinal dimension of the multi-level structure when the multi-level structure is in its erected configuration, and the attachment of all ligaments is such that the free intermediate portions of all ligaments in the multi-level structure are able to convert from an initial flat configuration to an erected configuration upon application of a force along the longitudinal dimension of the multi-level structure, thus converting the multi-level structure as a whole from an initial flat configuration into an erected configuration, wherein the erection is in the direction perpendicular to the longitudinal and the lateral dimension of the multi-level structure.

2. The multi-level structure of claim 1, wherein the multi-level structure further comprises a second intermediate layer provided between the first intermediate layer and the first outermost layer such that the structure comprises a third level.

3. The multi-level structure of claim 2, wherein the multi-level structure further comprises one or more additional intermediate layer(s), provided between the second intermediate layer and the first outermost layer such that the structure comprises a one or more additional levels.

4. The multi-level structure of any of the preceding claims, wherein one or more intermediate layers are discontinuous along the longitudinal layer dimension such that the intermediate layer(s) comprise(s) at least two sections, wherein one or more than one ligament is attached to each of the sections with one of their first and second ligament attachment regions.

5. The multi-level structure of any of the preceding claims, wherein the multilevel structure comprises one or more stop aid(s) which define(s) the maximum shifting of the first outermost layer relative to the second outermost layer along the longitudinal dimension in opposite directions when the force along the longitudinal dimension is continued to be applied, wherein the maximum shifting defined by the stop aid is less than the maximum shifting possible in the absence of such stop aid.

6. The multi-level structure of any of the preceding claims, wherein at least one intermediate layer is not joined to the first or second outermost layer other than indirectly via ligaments and/or indirectly by a stop aid.

7. The multi-level structure of any of the preceding claims, wherein at least one intermediate layer is joined directly to the first and/or second outermost layer and includes at least one leeway, the leeway enabling elongation of the multi-level structure along the longitudinal dimension.

8. The multi-level structure of claim 7, wherein the at least one leeway portion is provided in the at least one intermediate layer by a slack, an extensible material or a combination thereof.

9. The multi-level structure of any of the preceding claims, wherein the ligaments of one level differ from the ligaments of another level in bending stiffness, tensile strength, or both.

10. The multi-level structure of any of the preceding claims, wherein the ligaments of a level differ from each other in bending stiffness and/or tensile strength, preferably one or more ligaments which are closer to the lateral edges of the multi-level structure have a lower bending stiffness and/or lower tensile strength compared to one or more ligaments which are arranged in or towards the center of the multi-level structure along the longitudinal dimension.

11. The multi-level structure of any of the preceding claims, wherein the erected multi-level structure perpendicular to the longitudinal and lateral dimension is higher in the center of the multi-level structure along the longitudinal dimension compared to the areas being closer towards the lateral edges of the multi-level structure.

12. The multi-level structure of any of the preceding claims, wherein, in the erected multi-level structure, the ligaments of the different levels are aligned with each other, such that the position of the ligaments in one level coincide with the position of the ligaments in the one of more levels when viewed perpendicular to the longitudinal and lateral dimension.

13. The multi-level structure of any of the preceding claims, wherein, in the erected multi-level structure, the ligaments of the different levels are staggered versus each other, such that the position of the ligaments in one level does not coincide with the position of the ligaments in the one of more levels when viewed perpendicular to the longitudinal and lateral dimension.

14. The multi-level structure of any of the preceding claims, wherein the ligaments, in their erected configuration, either adopt a Z-like shape, a C-like shape, a T-like shape, or a Double-T-like shape.

15. The multi-level structure of any preceding claims, wherein all ligaments adopt a Z-like shape when the structure is in its erected configuration and each ligament has a first surface and a second surface, wherein the ligament's first surface is not facing towards the superjacent layer when the structure is in its initial flat configuration, and wherein the ligament's second surface is not facing towards the subjacent layer when the structure is in its initial flat configuration.

16. A disposable consumer product comprising at least one multilevel structure of any of the preceding claims.

17. The disposable consumer product of claim 16, wherein the disposable consumer product is an absorbent article, a wound dressing or a bandage.

18. The disposable consumer product of claim 17, wherein, in the multilevel structure, the ligaments of the level which is closest to the skin of the wearer when the product is in use, have a lower bending stiffness and/or lower tensile strength compared to ligaments of levels being farther from the skin of the wearer.

19. A flexible packaging comprising at least one multilevel structure of any of claims 1 to 15.

20. A disposable absorbent article comprising a multilevel structure of any of the claims 1 to15, wherein the absorbent article is selected from the group consisting of a diaper, a pant and a sanitary napkin, and wherein the multilevel structure is comprised by one or more of: a front waist feature, a back waist feature, one or two front ears, one or two back ears.

21. The absorbent article of claim 20, wherein the longitudinal dimension of the multilevel structure is substantially parallel to a lateral centerline of the absorbent article and wherein the lateral dimension of the multilevel structure is substantially parallel to the longitudinal centerline of the absorbent article.

22. A disposable consumer product or a flexible package, wherein the disposable consumer product or flexible package comprises a multilevel structure of any of claims 1 to 15, wherein areas of the first and second layer at or adjacent to the lateral edges of the multilevel structure are attached to the disposable consumer product or flexible package.

Fig. 1a

Fig. 1b

Fig. 2a

Fig. 2b

Fig. 3a

Fig. 3b

**Fig. 4**

Fig. 5a

Fig. 5b

Fig. 5c

Fig. 6a

Fig. 6b

Fig. 6c

Fig. 7a

Fig. 7b

Fig. 7c

**Fig. 8a**

**Fig. 8b**

**Fig. 9a**

**Fig. 9b**

**Fig. 10**

Fig. 11a

Fig. 11b

Fig. 11c

Fig. 12a

Fig. 12b

Fig. 12c

Fig. 13a

Fig. 13b

Fig. 13c

Fig. 14a

Fig. 14b

Fig. 15a

Fig. 15b

**Fig. 16a**

**Fig. 16b**

Fig. 17a

Fig. 17b

Fig. 18a

Fig. 18b

# Fig. 19

**Fig. 20**

**Fig. 21**

Europäisches
Patentamt

European
Patent Office

Office européen
des brevets

# EUROPEAN SEARCH REPORT

Application Number

EP 14 18 4265

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 3 834 074 A (SHIROUZU A) 10 September 1974 (1974-09-10) * Figures 2, 3, 10, 11 * ----- | 1-22 | INV. A61F13/49 A61F13/494 A61F13/56 |
| A,D | US 2007/093768 A1 (ROE DONALD C [US] ET AL) 26 April 2007 (2007-04-26) * the whole document * ----- | 1-22 | A61F13/00 B32B5/04 B32B7/00 B32B7/04 |
| A | GB 2 235 200 A (NAT RES DEV [GB] NAT RES DEV [GB]; BRITISH TECH GROUP [GB]) 27 February 1991 (1991-02-27) * Figures 3a-3d; page 2, line 30 - page 3, line 19 * ----- | 1-22 | ADD. A61F13/15 |
| A | US 2011/240194 A1 (SUMMERS JOSHUA D [US] ET AL) 6 October 2011 (2011-10-06) * figure 2 * ----- | 1-22 | |

TECHNICAL FIELDS
SEARCHED      (IPC)

A61F
B32B

The present search report has been drawn up for all claims

| Place of search | Date of completion of the search | Examiner |
|---|---|---|
| The Hague | 27 February 2015 | Barenbrug, Theo |

EPO FORM 1503 03.82 (P04C01)

## ANNEX TO THE EUROPEAN SEARCH REPORT
## ON EUROPEAN PATENT APPLICATION NO.

EP 14 18 4265

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2015

| Patent document cited in search report | | Publication date | Patent family member(s) | | Publication date |
|---|---|---|---|---|---|
| US 3834074 | A | 10-09-1974 | NONE | | |
| US 2007093768 | A1 | 26-04-2007 | CA | 2627625 A1 | 26-04-2007 |
| | | | CN | 101291643 A | 22-10-2008 |
| | | | EP | 1945163 A1 | 23-07-2008 |
| | | | JP | 2009511111 A | 19-03-2009 |
| | | | US | 2007093768 A1 | 26-04-2007 |
| | | | WO | 2007046069 A1 | 26-04-2007 |
| GB 2235200 | A | 27-02-1991 | CA | 2063396 A1 | 15-01-1991 |
| | | | EP | 0482058 A1 | 29-04-1992 |
| | | | GB | 2235200 A | 27-02-1991 |
| | | | JP | H04506638 A | 19-11-1992 |
| | | | WO | 9101210 A1 | 07-02-1991 |
| US 2011240194 | A1 | 06-10-2011 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007046069 A1
- US 3860003 A
- US 5221274 A
- US 5554145 A
- US 5569234 A
- US 5580411 A
- US 6004306 A